# EUROPEAN PATENT SPECIFICATION

(11) **EP 3 482 752 B1**
(45) Date of publication and mention of the grant of the patent: **25.03.2020**
(21) Application number: 18205072.4
(22) Date of filing: 08.11.2018
(51) Int. Cl.: A61K 31/05, A61K 31/357, A61K 36/03, A61P 9/10

(54) **AN ECKLONIA CAVA EXTRACT AND A PHARMACEUTICAL COMPOSITION FOR PREVENTING OR TREATING VASCULAR DISEASES COMPRISING THE SAME AS AN ACTIVE INGREDIENT**
ECKLONIA-CAVA-EXTRAKT UND PHARMAZEUTISCHE ZUSAMMENSETZUNG ZUR VORBEUGUNG ODER BEHANDLUNG VON GEFÄSSERKRANKUNGEN DAMIT ALS EIN WIRKSTOFF
EXTRAIT D'ECKLONIA CAVA ET COMPOSITION PHARMACEUTIQUE POUR LA PRÉVENTION OU LE TRAITEMENT DE MALADIES VASCULAIRES LE COMPRENANT COMME INGRÉDIENT ACTIF

(30) Priority: 14.11.2017 KR 20170151830; 04.04.2018 KR 20180039086
(43) Date of publication of application: 15.05.2019
(73) Proprietor: Gachon University of Industry-Academic Cooperation Foundation, Seongnam-si, Gyeonggi-do 13120 (KR); Jeju National University Industry-Academic Cooperation Foundation, Jeju-si, Jeju-do 63243 (KR)
(72) Inventor: BYUN, Kyunghee, 22009 Incheon (KR); SON, Myeongjoo, 21977 Incheon (KR); LEE, Hye Sun, 1442 Gyeonggi-do (KR); OH, Seyeon, 12213 Gyeonggi-do (KR); CHOI, Junwon, 04334 Seoul (KR); JEON, You-Jin, 63093 Jeju-do (KR)
(74) Representative: Abel & Imray

(56) References cited:
- DATABASE WPI Week 201273 Thomson Scientific, London, GB; AN 2012-N46760 XP002788362, "Sauce composition for spaghetti useful for preventing and/or treating hypertension and providing antioxidant effect, comprising Ecklonia cava enzyme extract", & KR 2012 0111057 A (UNIV GYEONGSANG IND ACAD COOP FOUND) 10 October 2012 (2012-10-10)
- KIM JEONG HWAN ET AL: "Protective efficacy of an Ecklonia cava extract used to treat transient focal ischemia of the rat brain", ANATOMY & CELL BIOLOGYKOREA (SOUTH)JUN 2012,, vol. 45, no. 2, 31 May 2012 (2012-05-31), pages 103-113, XP009510763, ISSN: 2093-3673, DOI: 10.5115/ACB.2012.45.2.103
- KANG SUNG-MYUNG ET AL: "Evaluation of antioxidant properties of a new compound, pyrogallol-phloroglucinol-6,6'-bieckol isolated from brown algae, Ecklonia cava", NUTRITION RESEARCH AND PRACTICEKOREA (SOUTH)DEC 2011,, vol. 5, no. 6, 30 November 2011 (2011-11-30), pages 495-502, XP009510759, ISSN: 2005-6168
- YOON JIN-SOO ET AL: "Dieckol, isolated from Ecklonia stolonifera, induces apoptosis in human hepatocellular carcinoma Hep3B cells", NATURAL MEDICINES - SHOYAKUGAKU ZASSHI, JAPANESE SOCIETY OF PHARMACOGNOSY, TOKYO, JP, vol. 67, no. 3, 30 June 2013 (2013-06-30), pages 519-527, XP009510760, ISSN: 1340-3443, DOI: 10.1007/S11418-012-0709-0
- LEE S H ET AL: "Cytoprotective effect of dieckol on human endothelial progenitor cells (hEPCs) from oxidative stress-induced apoptosis", FREE RADICAL RESEARCH, TAYLOR & FRANCIS, GB, vol. 47, no. 6-7, 30 June 2013 (2013-06-30), pages 526-534, XP009510762, ISSN: 1071-5762, DOI: 10.3109/10715762.2013.797080
- MOON HYE EUN ET AL: "Inhibitory activity of Ecklonia stolonifera and its isolated phlorotannins against Cu2+-induced low-density lipoprotein oxidation", FISHERIES SCIENCE, JAPANESE SOCIETY OF SCIENTIFIC FISHERIES, JP, vol. 78, no. 4, 30 June 2012 (2012-06-30), pages 927-934, XP009510761, ISSN: 0919-9268, DOI: 10.1007/S12562-012-0511-7
- KIM T H ET AL: "Ecklonia cava extracts inhibit lipopolysaccharide induced inflammatory responses in human endothelial cells", FOOD AND CHEMICAL TOXICOLOGY, PERGAMON, GB, vol. 48, no. 6, 1 June 2010 (2010-06-01), pages 1682-1687, XP027400132, ISSN: 0278-6915 [retrieved on 2010-06-01]
- LEE JI-HYEOK ET AL: "Preparative isolation and purification of phlorotannins fromEcklonia cavausing centrifugal partition chromatography by one-step", FOOD CHEMISTRY, vol. 158, 28 February 2014 (2014-02-28), pages 433-437, XP028867754, ISSN: 0308-8146, DOI: 10.1016/J.FOODCHEM.2014.02.112

## Description

### BACKGROUND OF THE INVENTION

### 1. Field of the Invention

The present invention relates to an Ecklonia cava extract and a pharmaceutical composition, comprising the same as an active ingredient, for use in the treatment of various vascular diseases.

### 2. Description of the Related Art

In the early 20th century, deaths from cardiovascular diseases accounted for less than 10% of all deaths, but recently death rates from these cardiovascular diseases accounted for about 50% in developed countries and about 25% in developing countries. The majority of cardiovascular diseases are atherosclerosis which is caused when blood vessels are damaged by various factors such as smoking and diabetes, or when vascular endothelial cell function is impaired. In the case of atherosclerosis, the generation of atherosclerotic plaques and the resulting thrombosis cause a serious problem by interrupting blood supply to each organ.

Atherosclerosis is a major cause of cardiovascular disease, and the incidence is rapidly increasing due to the rapid social and economic development and westernized lifestyle over the past 20 ∼ 30 years. Therefore, the importance of studies on pathogenesis, mechanism and treatment of such disease is gradually growing. Atherosclerosis is a disease which is developed when cytokines are secreted in endothelial cells and mononuclear cells by inflammatory reaction initiated from the oxidative damage of vascular endothelial cells, which causes migration and proliferation of smooth muscle cells in the endothelium and as a result blood circulation is inhibited by the formation of the vascular endothelial plaque.

The treatment methods for arterial disease developed so far are drug therapy, gene therapy, percutaneous transluminal coronary angioplasty and stenting (PTCA), and revascularization therapy of coronary artery bypass graft (CABG), among which revascularization therapy is widely used. The coronary artery regeneration technique such as coronary angioplasty or stenting has a problem that vascular restenosis occurs in about 40% of patients after 3 to 6 months after the treatment.

The mechanism of vascular restenosis is known as follows: when various cytokines and growth factors are generated and secreted in vascular endothelial cells damaged by a catheter equipped with a balloon, activated platelets or macrophages, the migration and proliferation of vascular smooth muscle cells are promoted. Then, the generation and secretion of extracellular matrix (ECM) are accelerated, leading to hyperplasia of the endothelium.

Recent studies have shown that the migration and proliferation of vascular smooth muscle cells (VSMCs) are important processes in the formation and progression of atherosclerosis. Particularly, matrix metallo-proteinases (MMPs), among the important factors related to the development and progression of atherosclerosis, are expressed in macrophages, vascular endothelial cells, and smooth muscle cells stimulated by cytokines involved in atherosclerosis. The increased proteolytic activity of MMPs degrades the extracellular matrix (ECM) around the smooth muscle cells, leading to cell migration into the vascular endothelium. MMPs bind to PDGF receptors present in cell membrane of vascular smooth muscle cells, which causes cells to move to the inner membrane from the middle membrane and at the same time causes cell proliferation, resulting in the formation of neointima in the vascular endothelium. These processes mediate the initial response of atherosclerosis by inducing the reduction of the internal diameter of the blood vessel and the blood flow dysfunction.

Therefore, vascular disease can be prevented or treated by inhibiting the migration and proliferation of smooth muscle cells. For example, arteriosclerosis or vascular restenosis can be prevented or treated by that.

To suppress arteriosclerosis or vascular restenosis, anti-platelet agents, anticoagulants, cholesterol biosynthesis inhibitors, and anti-allergic agents have been used so far. However, such inhibitors have various side effects such as inhibition of wound regeneration, vascular injury, hepatotoxicity, and kidney damage In addition to inhibiting thrombus formation. In particular, such a side effect as vascular injury can be a reason of vascular disease. Studies have been actively going on to develop or screen a material from various natural substances that have been confirmed to be safe in human in order to solve the problem of side effects and to suppress the development of arteriosclerosis or vascular restenosis. However, the results are not satisfactory, yet.

Based on that, the present inventors tried to develop a material originated from natural substances to treat vascular disease. And the present inventors confirmed that the Ecklonia cava extract was efficient in improving vascular disease.

### SUMMARY OF THE INVENTION

It is an object of the present invention to provide a pharmaceutical composition for the treatment of vascular disease.

It is further an object of the present invention to provide an Ecklonia cava extract for use in the treatment of a vascular disease.

To achieve the above objects, the present invention provides a pharmaceutical composition for treating vascular disease comprising an Ecklonia cava extract as an active ingredient, as per claim 1.

The present invention also provides an Ecklonia cava extract for use in the treatment of a vascular disease, as per claim 5.

### ADVANTAGEOUS EFFECT

The Ecklonia cava extract of the present invention was confirmed to have a medicinal effect on those factors involved in causing vascular diseases by the cell level experiments and the disease induced animal model experiments. Therefore, the Ecklonia cava extract of the present invention can be effectively used as an active ingredient of a pharmaceutical composition for the treatment of vascular diseases.

### BRIEF DESCRIPTION OF THE DRAWINGS

The application of the preferred embodiments of the present invention is best understood with reference to the accompanying drawings, wherein:
Figure 1 is a graph illustrating the yield (%) of the Ecklonia cava extract extracted by using 40%, 50% or 100% ethanol respectively in Example 1.
Figure 2 is a graph illustrating the vascular cell death inhibitory activity of the Ecklonia cava extract. To examine the activity, a mouse vascular cell death model was constructed by the treatment of 0.25 mM palmitic acid for 24 hours. Then, the animals were treated with three different Ecklonia cava extracts prepared in Example 1 (extracted with 40%, 50% or 100% ethanol, or extracted with hot water, 45% or 70% ethanol) respectively together with 100 µg/ml of palmitic acid, followed by investigation of the vascular cell death inhibitory activity.
Figure 3 is a graph illustrating the smooth muscle cell migration inhibitory activity of the Ecklonia cava extract. To examine the activity, a mouse vascular smooth muscle cell migration model was constructed by the treatment of 0.25 mM palmitic acid for 48 hours. Then, the animals were treated with three different Ecklonia cava extracts prepared in Example 1 (extracted with 40%, 50% or 100% ethanol, or extracted with hot water, 45% or 70% ethanol) respectively together with 100 µg/ml of palmitic acid, followed by investigation of the smooth muscle cell migration inhibitory activity.
Figure 4 is a graph illustrating the survival rate of vascular endothelial cells, investigated after treating the mouse vascular cell death model with the Ecklonia cava extract of the present invention (extracted with 50% ethanol) at the time of palmitic acid treatment at different concentrations (0, 25, 50, and 100 µg/ml).
Figure 5 is a graph illustrating the expression levels of PGI2 in the normal diet group (Control, NFD), the high fat diet group (HFD), and the experimental group (HFD-ECE) treated with the high fat diet and the Ecklonia cava extract.
Figure 6 is a graph illustrating the expression levels of E-selectin in the aorta of the normal diet group (Control, NFD), the high fat diet group (HFD), and the experimental group (HFD-ECE) treated with the high fat diet and the Ecklonia cava extract, which were analyzed by immunofluorescence staining and quantified.
Figure 7 is a graph illustrating the expression levels of ICAM-1 in the aorta of the normal diet group (Control, NFD), the high fat diet group (HFD), and the experimental group (HFD-ECE) treated with the high fat diet and the Ecklonia cava extract, which were analyzed by immunofluorescence staining and quantified.
Figure 8 is a graph illustrating the expression levels of VCAM-1 in the aorta of the normal diet group (Control, NFD), the high fat diet group (HFD), and the experimental group (HFD-ECE) treated with the high fat diet and the Ecklonia cava extract, which were analyzed by immunofluorescence staining and quantified.
Figure 9 is a graph illustrating the expression levels of vWF in the aorta of the normal diet group (Control, NFD), the high fat diet group (HFD), and the experimental group (HFD-ECE) treated with the high fat diet and the Ecklonia cava extract, which were analyzed by immunofluorescence staining and quantified.
Figure 10 is a graph illustrating the expression levels of ET-1 in the aorta of the normal diet group (Control, NFD), the high fat diet group (HFD), and the experimental group (HFD-ECE) treated with the high fat diet and the Ecklonia cava extract, which were analyzed by immunofluorescence staining and quantified.
Figure 11 is a graph illustrating the thickness of the smooth muscle layer of blood vessels in the aorta of the normal diet group (Control, NFD), the high fat diet group (HFD), and the experimental group (HFD-ECE) treated with the high fat diet and the Ecklonia cava extract, which were examined by immunofluorescence staining.
Figure 12 is a graph illustrating the ratio of endothelial cell layer to smooth muscle layer in blood vessels of the normal diet group (Control, NFD), the high fat diet group (HFD), and the experimental group (HFD-ECE) treated with the high fat diet and the Ecklonia cava extract.
Figure 13 is a graph illustrating the blood pressures of the normal diet group (Control, NFD), the high fat diet group (HFD), and the experimental group (HFD-ECE) treated with the high fat diet and the Ecklonia cava extract.
Figure 14 presents the results of HPLC performed with the ethyl acetate fraction (ECE) of the Ecklonia cava extract.
Figure 15 presents the results of CPC performed with the ethyl acetate fraction (ECE) of the Ecklonia cava extract.
Figure 16 presents the results of HPLC of pyrogallol-fluoroglucinol-6,6'-biexole (PPB) isolated from the ethyl acetate fraction (ECE) of the Ecklonia cava extract by using CPC.
Figure 17 presents the formula of pyrogallol-fluoroglucinol-6,6'-biexole (PPB) extracted as a single material.
Figure 18 presents the changes of fat cell size according to the administration of the Ecklonia cava extract (PPB) in the obesity-induced animal model of ob/ob mouse (leptin-deficient mouse).
Figure 19 presents the changes of fat accumulation in liver tissue according to the administration of PPB in the obesity-induced animal model of ob/ob mouse.
Figure 20 presents the changes of blood PAI-1 according to the administration of PPB in the obesity-induced animal model of ob/ob mouse.
Figure 21 presents the vascular endothelial cell death inhibitory effect according to the coadministration of PPB and palmitic acid on vascular endothelial cells.

### DESCRIPTION OF THE PREFERRED EMBODIMENTS

Hereinafter, the present invention is described in detail.

The following description should be understood as examples for the purpose of helping understanding of the present invention.

### Extract

The present invention provides a pharmaceutical composition for treating vascular disease comprising the Ecklonia cava extract as an active ingredient, wherein the Ecklonia cava extract is prepared by extraction using 45% ∼ 50% ethanol, and
the vascular disease is selected from the group consisting of vascular inflammatory diseases, vascular restenosis, vascular stenosis, arteriosclerosis, atherosclerosis, myocardial infarction, angina pectoris, hypertension, hypertensive heart disease and peripheral vascular stenosis.

Herein, the said Ecklonia cava indicates brown algae perennial seaweeds belonging to Phaeophyta, Laminariales, Alariaceae. Ecklonia cava lives mainly at the depth of about 10 m below the sea level in the coastal area of Jeju, Korea. It is also found in Japan. The length of Ecklonia cava is 1 ∼ 2 m and the stem is cylindrical. The base looks like roots.

The Ecklonia cava extract can be extracted from the whole or a part of Ecklonia cava plant by using an extraction solvent, for example, an inorganic or organic substance in a gaseous or liquid phase, or a mixture thereof, but not always limited thereto. As an example, the extract can be obtained by using a conventional extraction solvent which is exemplified by (a) C₁-C₄ anhydrous or hydrous lower alcohol (methanol, ethanol, propanol, butanol, n-propanol, iso-propanol and n-butanol, etc.) (b) a mixed solvent of the lower alcohol and water, (c) acetone, (d) ethyl acetate, (e) chloroform, (f) 1,3-butylene glycol, (g) hexane, (h) diethyl ether, (i) butyl acetate, or (j) water. Alternatively, the Ecklonia cava extract can be a supercritical extract of Ecklonia cava plant, for example, a supercritical CO₂ extract of Ecklonia cava.

In certain examples, Ecklonia cava extract can be an extract obtained by extracting the whole or at least a part of Ecklonia cava plant with water, C₁₋₂ lower alcohol or a mixture thereof.

In other examples (non-claimed), Ecklonia cava extract can be an extract obtained by extracting with 30% ∼ 100% ethanol, 40% ∼ 100% ethanol, 40% ∼ 90% ethanol, 40% ∼ 80% ethanol, 40% ∼ 70% ethanol, 40% ∼ 60% ethanol, 45% ∼ 60% ethanol.

The Ecklonia cava extract of the present invention is an extract obtained by extracting with 45% ∼ 50% ethanol, and preferably 50% ethanol.

In a preferred embodiment of the present invention, the Ecklonia cava extract can be extracted by the same manner as described in Example 1.

In cetain other examples (non-claimed), the Ecklonia cava extract can be extracted by using water, C₁₋₂ lower alcohol or a mixture thereof.

In other non-claimed examples, the Ecklonia cava extract extracted by using water can be extracted, for example, by using hot water.

In further non-claimed examples, the Ecklonia cava extract can be extracted with water at the temperature of 40 ∼ 100□, 50 ∼ 100□, 70∼ 100□, or 80 ∼ 100□.

In another preferred embodiment of the present invention, the Ecklonia cava extract can be extracted by the same manner as described in Example 2 below.

In another aspect of the present invention, the Ecklonia cava extract above can be understood as an active ingredient showing a therapeutic effect on vascular disease.

In a preferred embodiment of the present invention, the Ecklonia cava extract was confirmed to have the effect of inhibiting migration or proliferation of vascular smooth muscle cells. The Ecklonia cava extract of the present invention was also confirmed to have the effect of relieving thickening of blood vessel muscles and restoring the blood vessel wall to a normal level in the disease-induced animal model experiment. Thus, the Ecklonia cava extract of the present invention can be effectively used as an active ingredient of a pharmaceutical composition for treating vascular disease.

In another preferred embodiment of the present invention, the Ecklonia cava of the present invention was confirmed to increase the expression of prostaglandin (PGI2). In particular, PGI2 physiologically counteracts the vasoconstrictive factor thromboxane A2 (TxA2) (which acts as a vasoconstrictor) and relaxes blood vessels.

The extract of the present invention can treat the symptoms or diseases such as clogged blood vessels or hematological disorders, in connection with vascular system diseases, particularly. Therefore, the up-regulation of PGl2 induced by the Ecklonia cava extract of the present invention can bring the effect of relaxing blood vessels, so that the extract of the invention can be useful for treating vascular disease, particularly symptoms or diseases involved in vasoconstriction, blood vessel clogging and circulatory disturbance, etc.

Further, in another preferred embodiment of the present invention, the Ecklonia cava extract was confirmed to reduce the expressions of the intracellular adhesion proteins (E-selectin, ICAM-1, VCAM-1, and vWF). These proteins are the factors related to arteriosclerosis particularly, and at the same time the factors to cause inflammatory vascular disease. Therefore, the Ecklonia cava extract of the present invention is useful for the treatment of such vascular diseases as arteriosclerosis and inflammatory diseases.

In another preferred embodiment of the present invention, the Ecklonia cava extract of the present invention was confirmed to reduce the expression of endothelin-1 (ET-1). Endothelin-1 is a strong vasoconstrictor. So, it was suggested that the Ecklonia cava extract was able to prevent vasoconstriction. Therefore, the Ecklonia cava extract of the present invention can be effectively used for treating vascular disease, particularly symptoms or diseases involved in vasoconstriction, blood vessel clogging and circulatory disturbance, etc.

The Ecklonia cava extract of the present invention was confirmed to have at least one of those effects selected from the group consisting of preserving and improving blood vessel elasticity, suppressing abnormal proliferation of vascular visceral muscles, inhibiting endothelial cell damage, suppressing vascular outer wall fibrosis and preventing blood pressure increase. Therefore, the Ecklonia cava extract of the present invention can be provided as an active ingredient of a pharmaceutical composition for treating the vascular diseases described herein.

In certain examples, the vascular disease above can be understood as cardiovascular disease, in particular a disease in which the movement of blood circulation is inhibited, blocked or stenosed, or inflammatory diseases of blood vessels. Vascular diseases can be selected from abnormal proliferation of vascular smooth muscle cells, migratory or endothelial proliferative diseases, vascular inflammatory diseases, vascular restenosis, vascular stenosis, arteriosclerosis, atherosclerosis, heart failure, myocardial infarction, angina pectoris, arrhythmia, hypertension, hypertensive heart disease, congenital heart disease, stroke, and peripheral vascular stenosis.

Specific vascular diseases of the present invention include vascular inflammatory diseases, vascular restenosis, vascular stenosis, arteriosclerosis, atherosclerosis, myocardial infarction, angina pectoris, hypertension, hypertensive heart disease, and peripheral vascular stenosis

The vascular inflammatory disease herein is the inflammatory disease developed in blood vessels, which is exemplified by sepsis or septic shock.

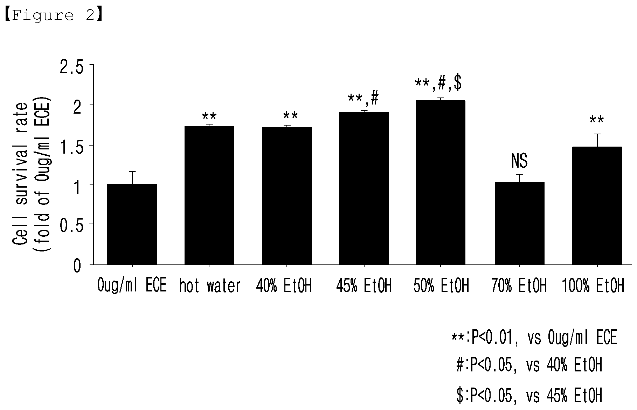

In another preferred embodiment of the present invention, as confirmed in the experimental example below, the ethanol extract, for example the ethanol extract described above, which was the Ecklonia cava extract prepared by using 50% ethanol, was confirmed to inhibit the migration of smooth muscle cells and mitigate thickening of vascular muscles, which was beneficiary to smooth blood flow, in an animal model. Therefore, the Ecklonia cava extract of the present invention can be effectively used as an active ingredient for the treatment of such diseases involved in the migration and proliferation of smooth muscle cells, and this result was confirmed by the experimental data shown in Figures 2, 3, 11, 12 and 13.

Therefore, the present invention provides a pharmaceutical composition comprising the Ecklonia cava extract of the present invention as an active ingredient for the treatment of one or more diseases selected from the group consisting of vascular diseases such as vascular inflammatory diseases, vascular restenosis, vascular stenosis, atherosclerosis, myocardial infarction, angina pectoris, hypertension, hypertensive heart disease, and peripheral vascular stenosis, as per claim 1.

The Ecklonia cava extract can be an additionally purified fraction or a fractional substance.

Herein, the fraction or the fractional substance above indicates a fraction or a fractional substance that can be used for the purpose of treating vascular diseases as shown in the experimental example below.

The fraction or the fractional substance thereof was confirmed to have one or more activities selected from the group consisting of suppressing the migration or proliferation of vascular smooth muscle cells, increasing the expression of prostaglandin (PGI2), reducing the expressions of intracellular adhesion proteins (E-selectin, ICAM-1, VCAM-1, and vWF) and reducing the expression of endothelin-1 (ET-1) or have one or more effects selected from the group consisting of preserving and improving blood vessel elasticity, suppressing abnormal proliferation of vascular visceral muscles, inhibiting endothelial cell damage, suppressing vascular outer wall fibrosis and preventing blood pressure increase, indicating the fraction or the fractional substance of the present invention is efficient in preventing, improving or treating vascular disease. The fraction or the fractional substance thereof is actually performing the same function as the Ecklonia cava extract of the invention and is just more separated and purified from the Ecklonia cava extract but still contains the same active ingredient.

The fraction or the fractional substance may mean a more isolated/purified form. For example, it can be a fraction obtained by filtering the Ecklonia cava extract with an ultrafiltration membrane having a constant molecular weight cut-off value or obtained by diverse chromatography (prepared for separation according to size, charge, hydrophobicity or affinity). Other fractions obtained by various purification methods that can be additionally performed can also be included in the fraction or the fractional substance of the Ecklonia cava plant.

In a preferred embodiment of the present invention, the Ecklonia cava extract can be an extract obtained by the preparation method comprising the following steps:
immersing the whole or at least a part of Ecklonia cava plant in a solvent; and
obtaining the Ecklonia cava extract from the solvent above, wherein the solvent is 45% ∼ 50% ethanol.

The raw material for the extraction can be prepared by cutting or additionally pulverizing the leaves, stems, roots or whole plants of Ecklonia cava plant, or at least a part thereof. In the stage of immersing, at least a part of the Ecklonia cava plant prepared as the above is dipped in a solvent, the extraction medium.

Some exemplary means of extraction are described hereinbelow:
The extraction medium which can be used in the preparation of the Ecklonia cava extract in addition to the above-mentioned solvent can be at least one kind of all types of gaseous or liquid inorganic or organic substances, and mixtures thereof. For example, the extraction medium can be selected from the group consisting of (a) C₁-C₄ anhydrous or hydrous lower alcohol (methanol, ethanol, propanol, butanol, n-propanol, iso-propanol and n-butanol, etc.) (b) a mixed solvent of the lower alcohol and water, (c) acetone, (d) ethyl acetate, (e) chloroform, (f) 1,3-butylene glycol, (g) hexane, (h) diethyl ether, (i) butyl acetate, and (j) water.

In the stage of obtaining the extract above, the active ingredient included in the extraction medium or solvent is obtained, and the active ingredient remaining in the residue can also be obtained. Particularly, any conventional method of separation or filtration, for example, separation or filtration of solid and liquid components can be used without limitation. For example, a filtration device can be used to separate the filtrate and the residue. The filtration herein can be a filtration step that is repeatedly extracted with a first order, a second order, or a higher order, and any method can be used without limitation as long as it can extract as many active ingredients from the residue as possible into the filtrate.

The filtrate itself can be used as the Ecklonia cava extract herein or it can be obtained in the form of an extract or a powder by further performing the steps of concentrating and lyophilizing. The extract can be dissolved or suspended again in a certain solvent according to the purpose of use.

For example, in order to obtain the active ingredient in a higher concentration, not only the preparation method of the fraction of the invention or the fractional substance thereof but also the conventional preparation method of the fraction or the fractional substance can be used without limitation.

In cetain examples, the Ecklonia cava plant can be extracted by using ethanol, for example 10% ∼ 100%, 20% ∼ 100%, 30% ∼ 100%, 40% ∼ 100%, 40% ∼ 90%, 40% ∼ 80%, 40% ∼ 70%, 40% ∼ 60%, 45% ∼ 60%. In an embodiment of the present invention, the Ecklonia cava plant is extracted by using 45% ∼50% ethanol, or preferably 50% ethanol.

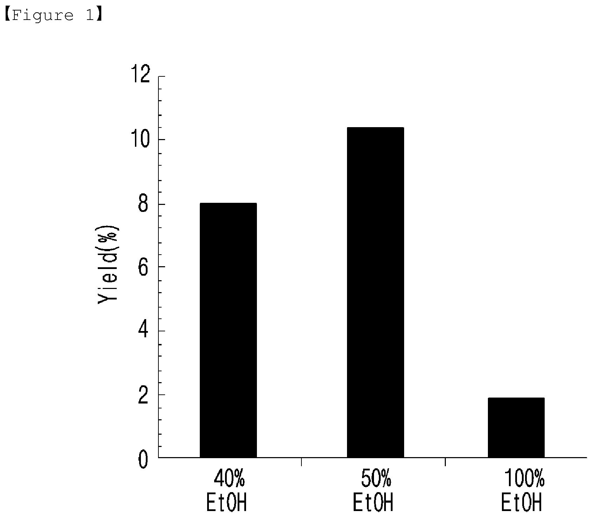

Ecklonia cava extracts were prepared by using different extraction solvents under different conditions, as shown in Figure 1. As a result, it was confirmed that the Ecklonia cava extracts were able to be prepared with high yield by using 40% ∼ 60%, 45% ∼ 60%, 45% ∼ 55% or 50% ethanol.

Further, as confirmed in the following experimental example, it is more preferred to use 40% ∼ 60% or 50% ethanol as an extraction solvent to produce the Ecklonia cava extract in a large scale or to increase industrial usability of the Ecklonia cava extract.

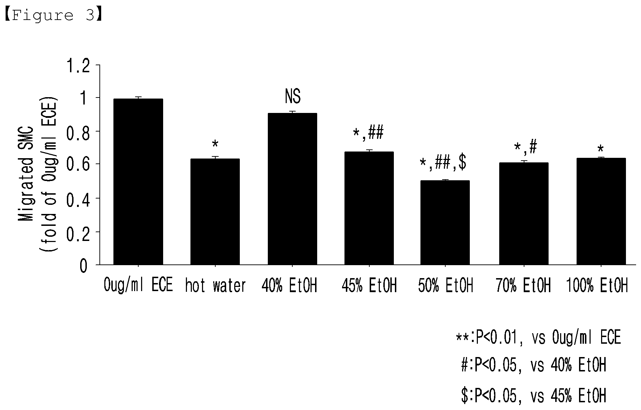

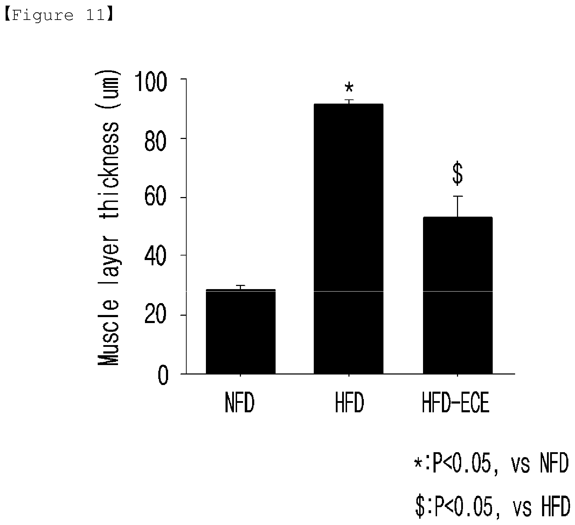

In a preferred embodiment of the present invention, the Ecklonia cava extract was confirmed to have the effect of suppressing the migration and proliferation of smooth muscle cells, as shown in Figure 3 presenting the experimental data. It was also confirmed that the thickness of vascular muscle layer was reduced by the extract as shown in Figures 11, 12 and 13.

To achieve the therapeutic effect on disease by suppressing the migration and proliferation of smooth muscle cells, it is preferred to use ethanol (of the concentration range descibed hereinabove) for the extraction.

Vascular diseases that can be treated by using the Ecklonia cava extract of the present invention is exemplified by abnormal proliferation of vascular smooth muscle cells, migratory or endothelial proliferative diseases.

For example, the abnormal proliferation of vascular smooth muscle cells, migratory or endothelial proliferative disease is one or more diseases selected from the group consisting of vascular restenosis, vascular stenosis, atherosclerosis, myocardial infarction, angina pectoris, hypertension, hypertensive heart disease, and peripheral vascular stenosis.

The extraction may be performed after adding the extraction solvent described above to the extraction sample (Ecklonia cava plant or at least a part thereof, pulverized product thereof, powder thereof, etc.) at the volume of 2 times, 5 times, 10 times, 20 times, 30 times, 40 times or 50 times the weight of the sample.

The extraction was performed at 0 ∼ 40□, 10 ∼ 40□, 20 ∼ 40□, 30 ∼ 40□, 20 ∼ 30□, or room temperature. The extraction time was not limited but preferably 10 minutes, 20 minutes, 30 minutes, 40 minutes, 1 hour, 2 hours, 5 hours, or 10 hours or more. For the extraction, the extraction medium and the extraction raw material were sufficiently mixed and could be stirred. Any method that can extract preferably the useful component from the extraction medium and the extraction solvent containing the same can be used to prepare the extract of the present invention.

The present invention provides the Ecklonia cava extract, as per claim 5.

In this invention, the surprising usability of the Ecklonia cava extract for vascular disease was confirmed.

Particularly, as shown in the Experimental Examples below and Figures attached in this invention, the Ecklonia cava extract of the present invention was confirmed to have the activities of suppressing the migration or proliferation of vascular smooth muscle cells, increasing the expression of prostaglandin (PGI2), reducing the expressions of intracellular adhesion proteins (E-selectin, ICAM-1, VCAM-1, and vWF) and reducing the expression of endothelin-1 (ET-1) in Experimental Examples 1 ∼ 5.

Therefore, it was confirmed that the Ecklonia cava extract of the present invention was efficient in preserving and improving blood vessel elasticity, suppressing abnormal proliferation of vascular visceral muscles, inhibiting endothelial cell damage, suppressing vascular outer wall fibrosis and preventing blood pressure increase.

The effect of the Ecklonia cava extract of the present invention was on the treatment of vascular disease, suggesting that the Ecklonia cava extract of the present invention is useful.

The present invention provides a pharmaceutical composition for treating vascular disease comprising the Ecklonia cava extract as an active ingredient, as per claim 1.

Examplary vascular disease can be understood as one or more diseases or symptoms selected from the group consisting of abnormal proliferation of vascular smooth muscle cells, migratory or endothelial proliferative diseases, vascular inflammatory diseases, vascular restenosis, vascular stenosis, atherosclerosis, heart failure, myocardial infarction, angina pectoris, arrhythmia, hypertension, hypertensive heart disease, congenital heart disease, stroke, and peripheral vascular stenosis.

The vascular diseases as per independent claims 1 and 5 are selected from the group consisting of vascular inflammatory diseases, vascular restenosis, vascular stenosis, atherosclerosis, myocardial infarction, angina pectoris, hypertension, hypertensive heart disease and peripheral vascular stenosis

The usability of the Ecklonia cava extract of the present invention in treating the diseases or symptoms described above is supported by the Experimental Examples and figures presented in this invention.

The Ecklonia cava extract of the present invention can treat the vascular disease described above by one or more activities selected from the group consisting of suppressing the migration or proliferation of vascular smooth muscle cells, increasing the expression of prostaglandin (PGI2), reducing the expressions of intracellular adhesion proteins (E-selectin, ICAM-1, VCAM-1, and vWF) and reducing the expression of endothelin-1 (ET-1).

The pharmaceutical composition comprising the Ecklonia cava extract, the fraction thereof or the active ingredient thereof of the present invention can be administered orally or parenterally and be used in general forms of pharmaceutical formulation. That is, the pharmaceutical composition of the present invention can be prepared for oral or parenteral administration by mixing with generally used diluents or excipients such as fillers, extenders, binders, wetting agents, disintegrating agents and surfactants.

The formulations for oral administration are exemplified by tablets, pills, hard/soft capsules, solutions, suspensions, emulsions, syrups, granules, elixirs, and troches, etc. These formulations can include diluents (for example, lactose, dextrose, sucrose, mannitol, sorbitol, cellulose, and/or glycine) and lubricants (for example, silica, talc, stearate and its magnesium or calcium salt, and/or polyethylene glycol) in addition to the active ingredient. Tablets can include binding agents such as magnesium aluminum silicate, starch paste, gelatin, methylcellulose, sodium carboxymethylcellulose and/or polyvinylpyrolidone, and if necessary disintegrating agents such as starch, agarose, alginic acid or its sodium salt or azeotropic mixtures and/or absorbents, coloring agents, flavours, and sweeteners can be additionally included thereto.

The pharmaceutical composition of the present invention can be administered by parenterally and the parenteral administration includes subcutaneous injection, intravenous injection, intramuscular injection and intrathoracic injection.

To prepare the composition as a formulation for parenteral administration, the Ecklonia cava extract, the fraction thereof or the active ingredient thereof of the present invention is mixed with a stabilizer or a buffering agent to produce a solution or suspension, which is then formulated as ampoules or vials. The composition herein can be sterilized and additionally contains preservatives, stabilizers, wettable powders or emulsifiers, salts and/or buffers for the regulation of osmotic pressure, and other therapeutically useful materials, and the composition can be formulated by the conventional mixing, granulating or coating method.

The effective dose of the Ecklonia cava extract, the fraction thereof or the active ingredient thereof of the present invention can be determined according to age, weight, gender, administration form, health condition, and severity of a disease. The dose is generally 0.1-1000 mg/day, preferably 1-500 mg/day, based on an adult subject having a body weight of 70 kg, which can be administered once or several times a day at a predetermined time interval according to the judgment of a doctor or a pharmacist.

An exemplary method for treating vascular disease comprising the step of administering a therapeutically effective dose of the Ecklonia cava extract, the fraction thereof or the active ingredient of the same to a subject is also described herein.

Herein, the vascular disease above is as described in this description.

The therapeutically effective dose above indicates the amount that can improve, treat, prevent or relieve of symptom or condition of a subject when it is administered *in vivo.* The amount above can vary from weight, age, gender, condition and family history of a subject. The treatment method determines the amount considering all the different conditions of subjects.

The "effective dose" indicates the amount that is efficient in treating disease.

The extract or composition of the present invention can be administered using any amount and any administration pathway effective for the treatment of disease. The exact amount required will vary by subject, depending on the species, age, general disease condition, severity of infection, specific drug in use, and administration pathway. The extract of the present invention can be formulated frequently in dosage unit form for ease of administration and uniformity of dosage. The term "dosage unit form" herein indicates a physically discrete unit of a formulation suitable for the subject to be treated as described in this description. The total daily dosage of the composition of the present invention can be determined by a patient's doctor within the scope of sound medical judgment.

The specific effective dosage level for any particular subject or organism depends on various factors containing the following: disease type and severity of a disease; activity of a specific active ingredient used; kind of composition used; age, weight, general health condition, gender and dietary of a subject; administration time, administration pathway, discharge rate of a specific active ingredient used; duration of treatment; single or co-treatment of a specific active ingredient with other drugs; and other factors generally known in the field of medicine.

The term "subject" indicates an animal, for example mammals, or human in this invention.

An exemplary health functional food composition for preventing or improving vascular disease comprising the Ecklonia cava extract as an active ingredient is also described herein.

Herein, the vascular disease can be one or more diseases selected from the group consisting of abnormal proliferation of vascular smooth muscle cells, migratory or endothelial proliferative diseases, vascular inflammatory diseases, vascular restenosis, vascular stenosis, atherosclerosis, heart failure, myocardial infarction, angina pectoris, arrhythmia, hypertension, hypertensive heart disease, congenital heart disease, stroke, and peripheral vascular stenosis.

In the Experimental Examples below, the Ecklonia cava extract, particularly a water extract and an ethanol extract, more specifically a hot water extract and a 40% ∼ 100% ethanol extract are preferably described as a possible active ingredient of a health functional food composition for the prevention or improvement of one or more diseases selected from the group consisting of such vascular diseases as abnormal proliferation of vascular smooth muscle cells, migratory or endothelial proliferative diseases, vascular inflammatory diseases, vascular restenosis, vascular stenosis, atherosclerosis, heart failure, myocardial infarction, angina pectoris, arrhythmia, hypertension, hypertensive heart disease, congenital heart disease, stroke, and peripheral vascular stenosis.

In other non-claimed examples, the fraction of the Ecklonia cava extract or the fractional substance separated therefrom can be provided as an active ingredient for a health functional food composition.

Herein, the health functional food composition can include, in addition to the active ingredient, the components that are generally added during the production of a food product, for example, proteins, carbohydrates, fats, nutrients, seasonings, and flavoring agents.

The carbohydrates above can be one of monosaccharides such as glucose and fructose, disaccharides such as maltose and sucrose, polysaccharides such as dextrin and cyclodextrin, and sugar alcohols such as xilytole, sorbitol and erythritol. Besides, natural sweetening agents (thaumatin, stevia extract, for example rebaudioside A, glycyrrhizin, etc.) and synthetic sweetening agents (saccharin, aspartame, etc.) can be included as a sweetening agent.

The exemplary health functional food compositions described herein can be formulated as a drink. In that case, it can additionally contain citric acid, liquid fructose, sugar, glucose, acetic acid, malic acid, juice, mulberry extract, jujube extract, and licorice extract in addition to the compound represented by formula 1 above, the Ecklonia cava extract comprising the same, the fraction thereof, or the ECE fraction thereof.

### Compound (pyrogallol-phloroglucinol-6,6'-bieckol, PPB)

Hereinafter, the present invention is described in detail.

The following description should be understood as examples for the purpose of helping understanding of the present invention.

The Ecklonia cava extract of the present invention preferably conatains a compound represented by formula 1 below, a stereoisomer thereof or a pharmaceutically acceptable salt thereof:

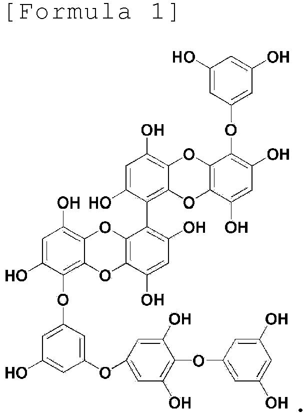

The compound represented by formula 1 can be named pyrogallol-phloroglucinol-6,6-bieckol (PPB).

The compound represented by formula 1 is pyrogallol-phloroglucinol-6,6-bieckol (PPB) which may be a single substance originated from Ecklonia cava, the stereoisomer thereof or the pharmaceutically acceptable salt thereof.

The present invention was introduced in order to solve the problems of side effects accompanied by the conventional synthetic drugs and to meet the request of the development of an efficient drug originated from a natural substance. The Ecklonia cava originated PPB is a natural substance originated compound, indicating that it is an active ingredient meeting the above requirements.

The PPB compound originated Ecklonia cava was confirmed to have an anti-obesity effect, indicating that this ingredient can be applied to treat metabolic disease.

The compound represented by formula 1 was confirmed to prevent blood coagulation and to reduce blood inflammation index and further to improve fibrinolysis related factors.

Therefore, the compound represented by formula 1 was confirmed to have an antithrombotic effect, and thus it can be applied to treat thrombotic disease.

The compound represented by formula 1 of the present invention was confirmed to protect vascular endothelial cells, to suppress vasoconstrictors and to relax blood vessels, suggesting that this compound was able to apply to treat vascular diseases.

Therefore, the compound represented by formula 1 of the present invention originated from Ecklonia cava and named pyrogallol-phloroglucinol-6,6-bieckol (PPB) can be applied to treat metabolic disease, vascular disease or thrombotic disease due to its anti-obesity effect, anti-thrombotic effect and vascular protective effect.

The anti-obesity effect shown in the PPB treated group, among the Ob/Ob mouse obesity induced animals, resulted in the decrease of fat tissue size and the decrease of accumulated fat in the liver tissue. As a result, body weight of the animal was reduced, suggesting that obesity was improved.

The extract of the present invention was confirmed to prevent vascular endothelial cell damage, to reduce the generation of fibrin in the outer wall of the blood vessel, to suppress the abnormal proliferation of vascular intramuscular layer and to improve the elasticity of blood vessels, suggesting that the compound was confirmed to have the effect of preventing, improving or treating vascular diseases.

The compound represented by formula 1 was originated from Ecklonia cava.

The compound represented by formula 1 is preferably originated from Ecklonia cava, more preferably originated from the Ecklonia cava extract or the fraction of the same.

Herein, the said Ecklonia cava indicates brown algae perennial seaweeds belonging to Phaeophyta, Laminariales, Alariaceae. Ecklonia cava lives mainly at the depth of about 10 m below the sea level in the coastal area of Jeju, Korea. The length of Ecklonia cava is 1 ∼ 2 m and the stem is cylindrical. The base looks like roots.

The compound represented by formula 1 above can be obtained from Ecklonia cava, the Ecklonia cava extract or the fraction of the same.

The Ecklonia cava extract can be an additionally purified fraction or a fractional substance.

Herein, the fraction or the fractional substance above indicates a fraction or a fractional substance that preferably contains at least a part of the compound represented by formula 1 so that it can be used for the purpose of treating vascular disease as shown in the experimental examples below.

The fraction or the fractional substance thereof was confirmed to have one or more activities selected from the group consisting of suppressing the migration or proliferation of vascular smooth muscle cells, increasing the expression of prostaglandin (PGI2), reducing the expressions of intracellular adhesion proteins (E-selectin, ICAM-1, VCAM-1, and vWF) and reducing the expression of endothelin-1 (ET-1) or have one or more effects selected from the group consisting of preserving and improving blood vessel elasticity, suppressing abnormal proliferation of vascular visceral muscles, inhibiting endothelial cell damage, suppressing vascular outer wall fibrosis and preventing blood pressure increase, indicating the fraction or the fractional substance of the present invention is efficient in preventing, improving or treating metabolic disease, vascular disease or thrombotic disease. The fraction or the fractional substance thereof is actually performing the same function as the Ecklonia cava extract of the invention and is just more separated and purified from the Ecklonia cava extract but still contains the same active ingredient.

The fraction or the fractional substance may mean a more isolated/purified form. For example, it can be a fraction obtained by filtering the Ecklonia cava extract with an ultrafiltration membrane having a constant molecular weight cut-off value or obtained by diverse chromatography (prepared for separation according to size, charge, hydrophobicity or affinity). Other fractions obtained by various purification methods that can be additionally performed can also be included in the fraction or the fractional substance of the Ecklonia cava plant.

Described herein is an examplary method for the preparation of the compound represented by formula 1 above, which comprises the following steps:
immersing the whole or at least a part of Ecklonia cava plant in a solvent;
obtaining the Ecklonia cava extract from the solvent above; and
separating and purifying the compound represented by formula 1 of claim 1 from the Ecklonia cava extract.

Herein, any solvent may be used as long as at least a part of the compound represented by formula 1 can be dissolved therein and extracted. For example, a conventional extraction solvent can be used and an extraction solvent suitable for the extraction of a general pharmaceutical composition or a health functional food composition is more preferred.

The solvent above can be a single solvent selected from the group consisting of water, methanol, ethanol, propanol, butanol, n-propanol, iso-propanol, n-butanol, acetone, ethyl acetate, 1,3-butylene glycol, hexane, diethyl ether and butyl acetate, or a mixed solvent comprising at least two of those solvents.

The solvent can be water, C₁₋₂ lower alcohol or a mixture thereof.

The exemplary preparation method of the compound represented by formula 1 above can additionally include a step of fractionating the Ecklonia cava extract by using water, C₁₋₄ alcohol, methylene chloride, chloroform, ethyl acetate, hexane, butanol, or a mixed solvent thereof.

The raw material for the extraction can be prepared by cutting or additionally pulverizing the leaves, stems, roots or whole plants of Ecklonia cava plant, or at least a part thereof. In the stage of immersing, at least a part of the Ecklonia cava plant prepared as the above is dipped in a solvent, the extraction medium. The extraction medium which can be used in the preparation of the Ecklonia cava extract in addition to the above-mentioned solvent can be at least one kind of all types of gaseous or liquid inorganic or organic substances, and mixtures thereof. For example, the extraction medium can be selected from the group consisting of (a) C₁-C₄ anhydrous or hydrous lower alcohol (methanol, ethanol, propanol, butanol, n-propanol, iso-propanol and n-butanol, etc.) (b) a mixed solvent of the lower alcohol and water, (c) acetone, (d) ethyl acetate, (e) chloroform, (f) 1,3-butylene glycol, (g) hexane, (h) diethyl ether, (i) butyl acetate, and (j) water.

In the stage of obtaining the extract above, the active ingredient included in the extraction medium or solvent is obtained, and the active ingredient remaining in the residue can also be obtained. Particularly, any conventional method of separation or filtration, for example, separation or filtration of solid and liquid components can be used without limitation. For example, a filtration device can be used to separate the filtrate and the residue. The filtration herein can be a filtration step that is repeatedly extracted with a first order, a second order, or a higher order, and any method can be used without limitation as long as it can extract as many active ingredients from the residue as possible into the filtrate.

The filtrate itself can be used as the Ecklonia cava extract herein or it can be obtained in the form of an extract or a powder by further performing the steps of concentrating and lyophilizing. The extract can be dissolved or suspended again in a certain solvent according to the purpose of use.

For example, in order to obtain the active ingredient in a higher concentration, not only the exemplary preparation method of the fraction or the fractional substance thereof but also the conventional preparation method of the fraction or the fractional substance can be used.

In a preferred embodiment of the present invention, the Ecklonia cava plant was extracted with 50% ethanol as the extraction solvent.

In addition, in order to mass-produce the Ecklonia cava extract or to improve the industrial usability of the Ecklonia cava extract comprising the compound represented by formula 1 above, the Ecklonia cava extract was prepared by using 50% ethanol.

In the exemplary preparation method of a compound represented by formula 1, the extraction was performed after adding the extraction solvent above to the extraction sample (Ecklonia cava plant or at least a part thereof, pulverized product thereof, powder thereof, etc.) at the volume of 2 times, 5 times, 10 times, 20 times, 30 times, 40 times or 50 times the weight of the sample.

In the meantime, it should be understood that the portions of the exemplary extraction method described herein may be easily modified and applied by those in the art, such as extraction time, extraction temperature, etc.

The extraction may be performed at 0 ∼ 40□, 10 ∼ 40□, 20 ∼ 40□, 30 ∼ 40□, 20 ∼ 30□, or room temperature. The extraction time is preferably 10 minutes, 20 minutes, 30 minutes, 40 minutes, 1 hour, 2 hours, 5 hours, or 10 hours or more. For the extraction, the extraction medium and the extraction raw material were sufficiently mixed and could be stirred.

An exemplary pharmaceutical composition for treating metabolic disease comprising the compound represented by formula 1 above, the stereoisomer thereof or the pharmaceutically acceptable salt thereof as an active ingredient is also described herein.

It was confirmed that the compound represented by formula 1 above was able to reduce the number of adipocytes and the fat accumulation in the liver significantly. Therefore, considering such an anti-obesity effect of the compound, the compound represented by formula 1, the stereoisomer thereof or the pharmaceutically acceptable salt thereof can be provided as an active ingredient for the prevention, improvement or treatment of metabolic disease.

The metabolic disease above includes all the diseases classified into metabolism related diseases, which are exemplified by metabolic syndrome, visceral fat syndrome, metabolic diseases caused by hypereutrophy due to overeating, heavy drinking and lack of exercise, metabolic diseases caused by genetic factors, metabolic diseases caused by aging factors, metabolic diseases resulting from excessive accumulation of triglycerides, insulin resistance, hyperglycemia, hyperlipoproteinemia, hypertension, cardiovascular disease, atherosclerosis, myocardial infarction, or other metabolic diseases causing the status such as obesity.

Preferably, the pharmaceutical composition for treating vascular disease comprises the compound represented by formula 1 above as an active ingredient.

In a preferred embodiment of the present invention, the vascular disease can be understood as such disease that can be prevented, improved, relieved or treated by the activities of the ECE fraction comprising the compound represented by formula 1 of the invention to reduce blood fibrinogen, to reduce blood plasminogen activator inhibitor-1, to increase the expression of prostaglandin (PGI2), to reduce the expressions of intracellular adhesion proteins (E-selectin, ICAM-1, VCAM-1, and vWF) and to reduce the expression of endothelin-1 (ET-1), as shown in the following experimental examples and figures attached in this invention.

In another preferred embodiment of the present invention, the vascular disease above can be understood as such disease in which the compound represented by formula 1 of the present invention inhibits thrombogenesis, preserves and improves blood vessel elasticity, inhibits abnormal proliferation of vascular visceral muscles, inhibits endothelial cell damage, suppresses vascular outer wall fibrosis and prevents blood pressure increase.

In a preferred embodiment of the present invention, the vascular disease can be selected from the group consisting of vascular inflammatory diseases, vascular restenosis, vascular stenosis, atherosclerosis, myocardial infarction, angina pectoris, hypertension, hypertensive heart disease, and peripheral vascular stenosis.

An exemplary pharmaceutical composition for preventing or treating thrombotic disease comprising the compound represented by formula 1 above, the stereoisomer thereof or the pharmaceutically acceptable salt thereof as an active ingredient is also described herein.

The thrombotic disease can be understood as a disease caused by thrombosis in blood circulation. The compound represented by formula 1 of the present invention can be understood to inhibit thrombosis, to prevent blood coagulation, to prevent platelet aggregation or to dissolve the generated thrombus, and therefore it is understood that the compound of the invention has the effect of preventing, improving, or treating thrombotic disease.

The thrombotic disease can be acute myocardial infarction, ischemic stroke, hemorrhagic stroke, deep vein thrombosis, lower limb edema, acute peripheral arterial occlusion, deep vein thrombosis, portal vein thrombosis, acute renal vein occlusion, cerebral sinus thrombosis, angina pectoris, cerebral infarction or central retinal vein occlusion.

The pharmaceutical composition comprising the compound represented by formula 1 can be administered orally or parenterally and be used in general forms of pharmaceutical formulation. That is, the pharmaceutical composition can be prepared for oral or parenteral administration by mixing with generally used diluents or excipients such as fillers, extenders, binders, wetting agents, disintegrating agents and surfactants.

The formulations for oral administration are exemplified by tablets, pills, hard/soft capsules, solutions, suspensions, emulsions, syrups, granules, elixirs, and troches, etc. These formulations can include diluents (for example, lactose, dextrose, sucrose, mannitol, sorbitol, cellulose, and/or glycine) and lubricants (for example, silica, talc, stearate and its magnesium or calcium salt, and/or polyethylene glycol) in addition to the active ingredient. Tablets can include binding agents such as magnesium aluminum silicate, starch paste, gelatin, methylcellulose, sodium carboxymethylcellulose and/or polyvinylpyrolidone, and if necessary disintegrating agents such as starch, agarose, alginic acid or its sodium salt or azeotropic mixtures and/or absorbents, coloring agents, flavours, and sweeteners can be additionally included thereto.

The pharmaceutical composition can be administered by parenterally and the parenteral administration includes subcutaneous injection, intravenous injection, intramuscular injection and intrathoracic injection.

To prepare the compound represented by formula 1 as a formulation for parenteral administration, the compound is mixed with a stabilizer or a buffering agent to produce a solution or suspension, which is then formulated as ampoules or vials. The composition herein can be sterilized and additionally contains preservatives, stabilizers, wettable powders or emulsifiers, salts and/or buffers for the regulation of osmotic pressure, and other therapeutically useful materials, and the composition can be formulated by the conventional mixing, granulating or coating method.

The effective dose of the compound represented by formula 1 or the pharmaceutical composition can be determined according to age, weight, gender, administration form, health condition, and severity of a disease. The dose is generally 0.1-1000 mg/day, preferably 1-500 mg/day, based on an adult subject having a body weight of 70 kg, which can be administered once or several times a day at a predetermined time interval according to the judgment of a doctor or a pharmacist.

The efficient usability of the compound represented by formula 1 above, that is pyrogallol-phloroglucinol-6,6-bieckol (PPB), in the treatment of diseases or symptoms above is supported by the experimental examples and figures of the present invention.

### Example 1: Preparation of Ecklonia cava extract

The following experiment was performed to prepare the Ecklonia cava extracts, including extracts of the present invention.

Particularly, Ecklonia cava powder was provided as an extraction raw material from Aqua Green Technology (Jeju, Korea). The extraction was performed under the different extraction conditions as described below, but using the same volume of the extraction raw material (2.5 g of Ecklonia cava powder) and the solvent (100 ml).
Condition 1: extraction with 40% ethanol (room temperature, 1 hr)
Condition 2: extraction with 45% ethanol (room temperature, 1 hr)
Condition 3: extraction with 50% ethanol (room temperature, 1 hr)
Condition 4: extraction with 70% ethanol (room temperature, 1 hr)
Condition 5: extraction with 100% ethanol (room temperature, 1 hr)

Each extract prepared by the different extraction methods performed under the different conditions was centrifuged (RPM: 10,000, 10 min, 4), followed by filtering to obtain supernatant. The obtained supernatant was concentrated under reduced pressure, followed by freeze-drying to give powders.

### Example 2: Preparation of Ecklonia cava extract

Ecklonia cava extract was prepared by using the same raw material as in Example 1, but using 100 hot water as an extraction solvent instead of ethanol.

### Experimental Example 1: Identification of optimum extraction condition of Ecklonia cava extract

To improve the industrial usability of the Ecklonia cava extract, the optimum extraction conditions of the Ecklonia cava extract were compared and evaluated as follows.

Particularly, the weight of the final Ecklonia cava extract powders obtained under the different extraction conditions as shown in Example 1 was measured to calculate the extraction yields, which were compared for the evaluation in order to determine the optimum extraction condition for the Ecklonia cava extract of the present invention.

The extraction yields calculated from the three different conditions (40%, 50% and 100% ethanol) are presented in Figure 1.

As shown in Figure 1, it was confirmed that the yield of the Ecklonia cava extract was highest when 50% ethanol was used as an extraction solvent (room temperature, 1 hr).

Therefore, it was confirmed that 50% ethanol was most preferred as an extraction solvent in order to increase the industrial usability of the Ecklonia cava extract or to provide the Ecklonia cava extract in a large industrial scale.

### Experimental Example 2: Evaluation of vascular endothelial cell death inhibitory activity of Ecklonia cava extract according to extraction conditions

To investigate the pharmaceutical efficacy of the Ecklonia cava extract of the present invention on vascular disease, the following experiment was performed to evaluate the vascular endothelial cell death inhibitory activity of the Ecklonia cava extract of the invention.

### <2-1> Vascular endothelial cell culture

First, mouse-derived vascular endothelial cells (SVEC4-10) in DMEM (Dulbecco's Modified Eagle Medium) supplemented with 10% fetal bovine serum and 1% antibiotics (penicillin/streptomycin) were cultured in a 37 , 5% CO₂ incubator.

To obtain sufficient cells, the cells were cultured in 100 Ø plastic dishes. When the cells were cultured until the bottom of the dish was covered 80% by the cells, the adhered cells were detached using trypsin/EDTA solution and then sub-cultured.

### <2-2> Construction of vascular endothelial cell death model

Mouse-derived vascular endothelial cells (SVEC4-10) were treated with 0.5 mM palmitic acid for 24 hours to construct a cell model (*in vitro*) similar to a high fat diet animal model (*in vivo*).

### <2-3> Evaluation of pharmaceutical efficacy of Ecklonia cava extract according to extraction conditions

In the course of constructing the vascular endothelial cell death model in Example <2-2>, three different Ecklonia cava extracts (100 ug/ml) prepared in Example 1 were treated thereto simultaneously with the treatment of 0.5 mM palmitic acid. 24 hours later, MTT assay was performed to investigate the number of survived vascular endothelial cells and the pharmaceutical efficacy of the Ecklonia cava extract of the present invention was evaluated. The results are shown in Figure 2.

As shown in Figure 2, it was confirmed that the survival rate of vascular endothelial cells was significantly increased in all of the Ecklonia cava extract treated groups of Example 1 and Example 2, compared with the non-treated control group.

Therefore, it was confirmed that the Ecklonia cava extract of the present invention had a medicinal effect of treating vascular disease. Thus, the Ecklonia cava extract above can be provided as an active ingredient of a pharmaceutical composition for vascular disease.

### Experimental Example 3: Evaluation of vascular smooth muscle cell migration inhibitory activity of Ecklonia cava extract according to extraction conditions

To investigate the pharmaceutical efficacy of the Ecklonia cava extract of the present invention on vascular disease, the following experiment was performed to evaluate the vascular smooth muscle cell migration inhibitory activity of the Ecklonia cava extract of the invention.

### <3-1> Mouse vascular smooth muscle cell culture

First, mouse-derived vascular smooth muscle cells (MOVAS) in DMEM (Dulbecco's Modified Eagle Medium) supplemented with 10% fetal bovine serum and 1% antibiotics (G418) were cultured in a 37□, 5% CO₂ incubator.

To obtain sufficient cells, the cells were cultured in 100 Ø plastic dishes. When the cells were cultured until the bottom of the dish was covered 80% by the cells, the adhered cells were detached using trypsin/EDTA solution and then sub-cultured.

### <3-2> Construction of vascular smooth muscle cell migration model

To construct a cell migration model used in transwell migration assay, the mouse originated vascular smooth cells (MOVAS) were cultured in the upper part of a transwell.

### <3-3> Evaluation of pharmaceutical efficacy of Ecklonia cava extract according to extraction conditions

Transwell migration assay was performed by using the cell migration model constructed in Example <3-2>. Particularly, the model was treated with 0.25 mM palmitic acid for 48 hours, and then the number of the cells migrated to the lower part was counted. The Ecklonia cava extracts (100 ug/ml) prepared under three different conditions in Example 1 were treated with palmitic acid for 48 hours thereto and MTT assay was performed to confirm the vascular smooth muscle cell migration. Based on that, the pharmaceutical efficacy of the Ecklonia cava extract of the present invention was evaluated and the results are shown in Figure 3.

As shown in Figure 3, a significant smooth cell migration inhibitory effect was observed in all experimental groups treated with the different Ecklonia cava extracts prepared respectively in Example 1 (ethanol extract) and Example 2 (hot water extract), compared with the non-treated control group. In particular, the inhibition of vascular smooth cell migration was excellent in those groups treated with the Ecklonia cava hot water extract of Example 2 and 45%, 50%, 70% and 100% ethanol extracts of Example 1.

The inhibition of vascular smooth cell migration was most significant in the group treated with Ecklonia cava 50% ethanol extract.

Therefore, the Ecklonia cava extract of the present invention can be provided as an active ingredient of a pharmaceutical composition for the treatment of vascular disease. In particular, the Ecklonia cava extract of the invention can be provided as a useful active ingredient that is particularly efficient in treating vascular diseases related to migration or proliferation of smooth muscle cells.

### Experimental Example 4: Evaluation of cell viability in vascular endothelial cell death model

To investigate the pharmaceutical efficacy of the Ecklonia cava extract of the present invention on vascular disease, the following experiment was performed to evaluate the cell viability in a vascular endothelial cell death model

### <4-1> Construction of vascular endothelial cell death model

Mouse-derived vascular endothelial cells (SVEC4-10) were treated with 0.25 mM palmitic acid for 24 hours to construct a cell model (*in vitro*) similar to a high fat diet animal model (*in vivo*).

### <4-2> Evaluation of pharmaceutical efficacy of Ecklonia cava extract

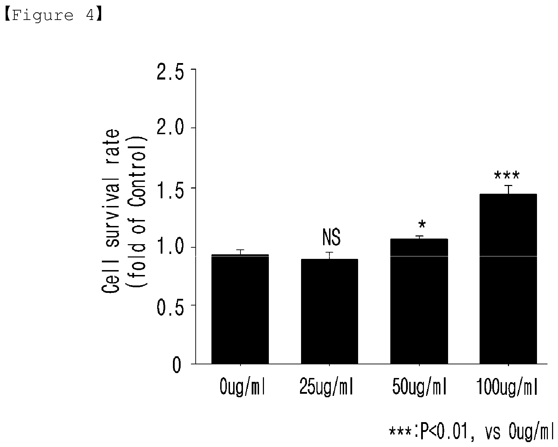

In the course of constructing the vascular endothelial cell death model in Example <4-1>, the Ecklonia cava 50% ethanol extract was treated thereto with 0.25 mM palmitic acid for 24 hours at different concentrations of 0 ug/ml, 25 ug/ml, 50 ug/ml and 100 ug/ml. 24 hours later, MTT assay was performed to confirm the number of survived vascular endothelial cells, and the cell viability was analyzed. The results are shown in Figure 4.

As shown in Figure 4, the cell survival rate of the group treated with the Ecklonia cava 50% ethanol extract at the concentration of 25 ug/ml was not much different from that of the non-treated control group. However, the cell survival rate was significantly increased in the groups treated with the Ecklonia cava 50% ethanol extract at the concentrations of 50 ug/ml and 100 ug/ml.

Therefore, it was confirmed that the Ecklonia cava extract of the present invention had a preventive effect on vascular endothelial cell death. Thus, the Ecklonia cava extract of the invention can be provided as an active ingredient of a pharmaceutical composition for treating the diseases mentioned in this invention including vascular disease.

### Experimental Example 5: Evaluation of pharmaceutical efficacy in disease induced animal model

The following experiment was performed to evaluate the pharmaceutical efficacy of the Ecklonia cava extract of the present invention in a disease induced animal model

### <5-1> Construction of vascular disease animal model

A vascular disease animal model was constructed by high fat diet as follows.

Particularly, the management and use of laboratory animals were carried out according to the protocol reviewed and approved by Institutional Animal Care and Use Committee, Lee Gil Ya Cancer and Diabetes Institute, Gachon University under the guidelines of Association for Assessment and Accreditation of Laboratory Animal Care (AAALAC). 8-week-old C57BL/6N mice (20-25g) were selected in order to investigate the effect of the Ecklonia cava extract on the body weight and blood pressure increased by 45% high fat diet (cat. D12451. Research diet) for 8 weeks.

The mice were adapted to the animal room environment for 1 week and then divided into the normal control diet group and the high fat diet group. Particularly, the mice were divided into three groups: The normal diet group (NFD) was fed with control diet for 8 weeks, the high fat diet group (HFD) was fed with high fat diet for 8 weeks, and the experimental group (HFD-ECE) was fed with high fat diet for the first 4 weeks and then co-treated with high fat food together with the Ecklonia cava extract (70 mg/kg) for the rest 4 weeks.
(1) normal diet group (NFD)
(2) high fat diet group (HFD)
(3) Ecklonia cava extract treated experimental group (HFD-ECE)

### <5-2> Measurement of blood pressure

Blood pressure of the animal model was measured by using tail cuff method a day before sacrifice.

The mice were placed in a holder after heating for 10 minutes on a warm plate at 37. A compression pad and a blood flow detector were attached on the tail, and systolic blood pressure and diastolic blood pressure of the tail artery were measured using a blood pressure measuring device (CODA-HT8, Kent Scientific Corporation). The mice were adapted to the warm plate and the holder for 6 days. Blood pressure was measured 5 times in a stationary state, which was considered as one cycle, and the measurement of blood pressure was repeated 3 ∼ 4 cycles and the mean values were calculated.

### <5-3> Immunohistochemical staining

Paraffin-embedded tissue block was thin-sectioned and the sections were attached on coating slides. The slides were dipped in xylene for 5 minutes three times and then dipped in 100%, 95%, 90%, 80% and 70% alcohol stepwise for 1 minute each for deparaffinization. For antibody recovery, the slides were dipped in an antibody recovery solution and treated with high frequency for 3 minutes. The primary antibody was diluted in PBS containing 2% normal horse serum at the concentration recommended by the antibody company, followed by reaction at 4 for 2 days. After reacting for 2 days, the slides were washed with PBS three times for 10 minutes each, and reacted with the secondary antibody conjugated with Alexa 488 (green) at room temperature for 1 hour. To observe the nuclei of the tissue, the slides were treated with DAPI (Sigma) for 3 minutes. The slides were washed with PBS three times for 10 minutes each. Each slide was covered by a cover glass, followed by observation.

### <5-4> Measurement of prostaglandin

Prostaglandin (PGI2) physiologically counteracts the vasoconstrictive factor thromboxane A2 (TxA2) (which acts as a vasoconstrictor) and relaxes blood vessels. Therefore, the changes of PGl2 expression can be a useful index of the evaluation of a pharmaceutical efficacy of the Ecklonia cava extract of the present invention on vascular disease. So, the expression of PGl2 in a high fat diet induced circulatory disturbance animal model was measured.

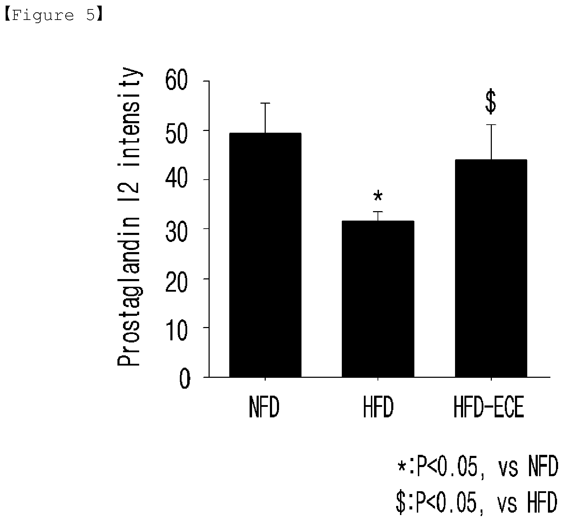

Particularly, the expression of PGl2 was measured in (1) the normal diet group (NFD), (2) the high fat diet group (HFD) and (3) the Ecklonia cava extract treated experimental group (HFD-ECE), and the results are shown in Figure 5.

As shown in Figure 5, the PGl2 expression was significantly reduced in the aorta of the HFD group but was significantly increased in the Ecklonia cava extract treated experimental group compared with the HFD group.

Therefore, it was confirmed that the Ecklonia cava extract of the present invention can be provided as an active ingredient of a pharmaceutical composition for the treatment of vascular disease.

### <5-5> Evaluation of changes in vascular endothelial function

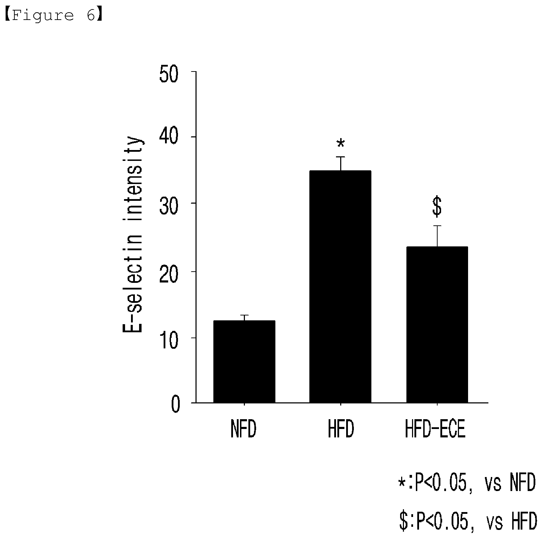

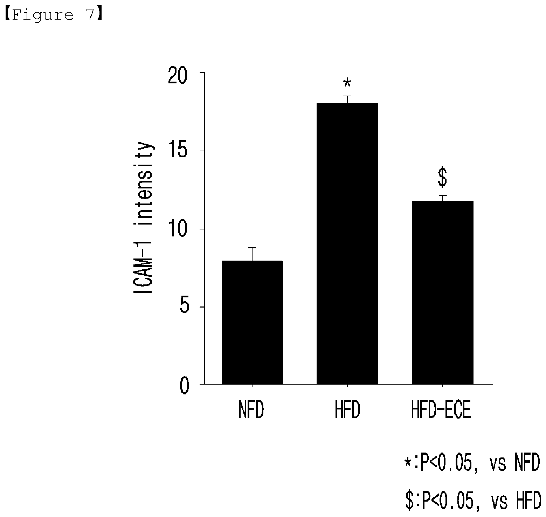

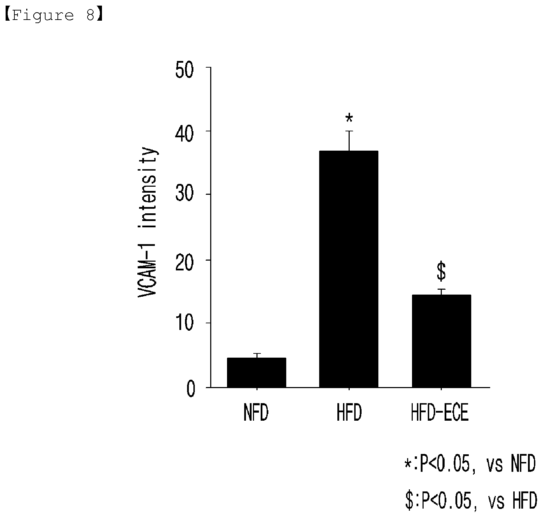

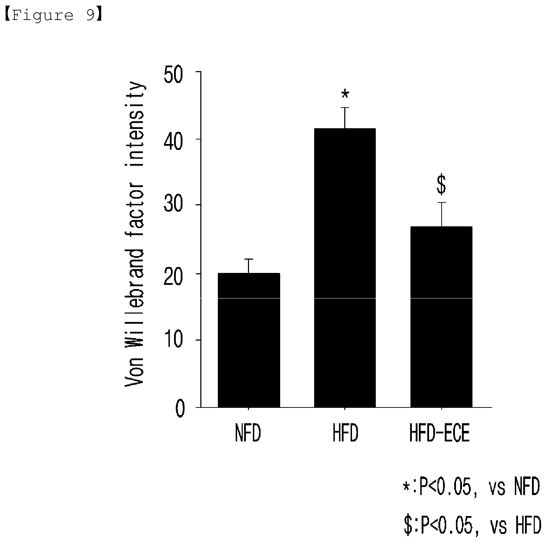

The expression patterns of prostaglandin (PGI2) and intracellular adherent proteins (E-selectin, ICAM-1, VCAM-1 and vWF) were investigated in (1) the normal diet group (NFD), (2) the high fat diet group (HFD) and (3) the Ecklonia cava extract treated experimental group (HFD-ECE), from which the changes in vascular endothelial function were evaluated. The results are shown in Figures 6, 7, 8 and 9.

As shown in Figures 6, 7, 8 and 9, when the adherent protein expression was confirmed in the NFD group and the HFD group, the expression of PGI2 was increase in the HFD group. However, the expression of PGI2 was suppressed in the HFD-ECE group, compared with the HFD group.

Therefore, it was confirmed that the Ecklonia cava extract of the present invention can be provided as an active ingredient of a pharmaceutical composition for the treatment of vascular disease.

### <5-6> Evaluation of inhibitory effect on vasoconstrictor that regulates blood vessel elasticity

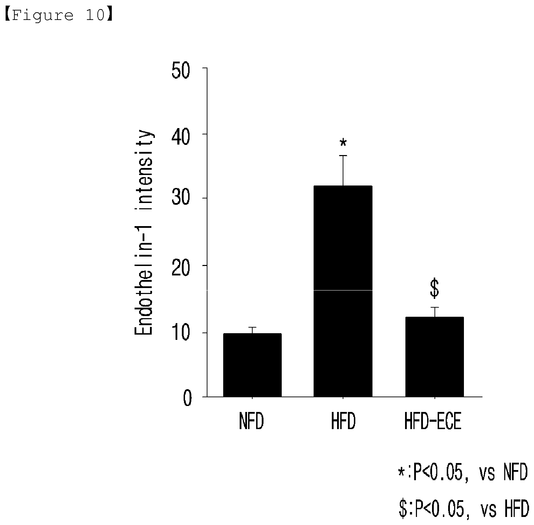

The expression of endothelin-1 (ET-1) relating to Vasoconstriction was investigated in (1) the normal diet group (NFD), (2) the high fat diet group (HFD) and (3) the Ecklonia cava extract treated experimental group (HFD-ECE), and the results are shown in Figure 10.

As shown in Figure 10, the expression of ET-1 in the Ecklonia cava extract treated experimental group was reduced 2.5 times the ET-1 expression in the HFD group.

Therefore, it was confirmed that the Ecklonia cava extract of the present invention maintained the blood vessel elasticity by reducing the expression of ET-1.

### <5-7> Observation of changes of vascular visceral muscle cells and blood pressure

Once vascular endothelial cells are damaged, vascular visceral muscle cells are abnormally deformed, resulting in abnormal cell growth and abnormal elasticity regulation. Such changes make the blood vessel thicker and reduce the elasticity of blood vessels, being a reason of vascular disease.

Thus, the changes of vascular visceral muscle cells in (1) the normal diet group (NFD), (2) the high fat diet group (HFD) and (3) the Ecklonia cava extract treated experimental group (HFD-ECE) were observed and the results are shown in Figure 11.

As shown in Figure 11, the thickness of vascular visceral muscle cells in the HFD group was increased 2.5 times compared with that of the NFD group but decreased 1.8 times in the HFD-ECE group.

Therefore, it was confirmed that the Ecklonia cava extract of the present invention inhibited the abnormal proliferation of vascular visceral muscle cells which are important in regulating blood vessel elasticity (Figure 11).

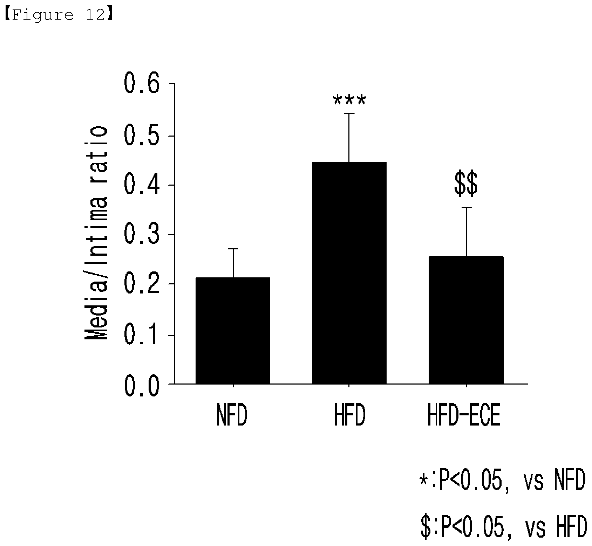

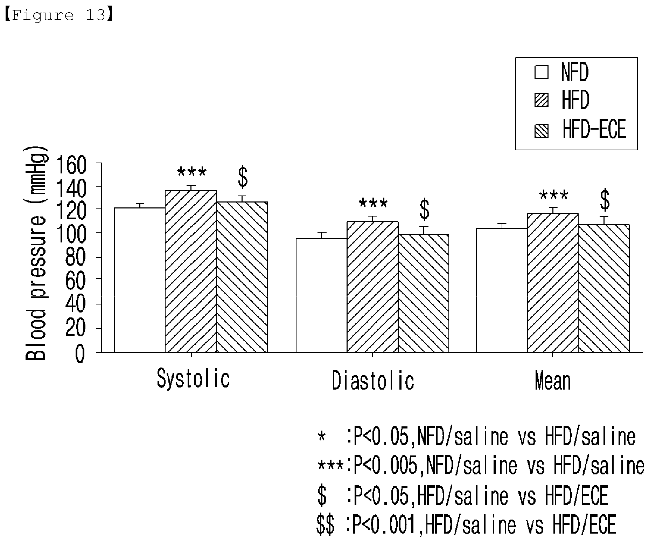

By such an inhibitory effect, the increase of blood pressure was reduced, indicating circulatory disturbance was improved (Figure 12). Further, the systolic and diastolic blood pressures and pulse pressure in the HFD group were significantly increased, compared with the normal control group. However, the systolic and diastolic blood pressures and pulse pressure in the Ecklonia cava extract treated group were significantly reduced (Figure 13).

Therefore, the Ecklonia cava extract of the present invention was confirmed to have the activities of inhibiting endothelial cell damage, suppressing vascular outer wall fibrosis and suppressing abnormal proliferation of vascular visceral muscles, so that the blood vessel elasticity was improved.

As shown in the Experimental Examples above and Figures attached in this invention, the Ecklonia cava extract of the present invention was confirmed to have the activities of suppressing the migration or proliferation of vascular smooth muscle cells, increasing the expression of prostaglandin (PGI2), reducing the expressions of intracellular adhesion proteins (E-selectin, ICAM-1, VCAM-1, and vWF) and reducing the expression of endothelin-1 (ET-1).

Therefore, it was confirmed that the Ecklonia cava extract of the present invention was efficient in preserving and improving blood vessel elasticity, suppressing abnormal proliferation of vascular visceral muscles, inhibiting endothelial cell damage, suppressing vascular outer wall fibrosis and preventing blood pressure increase.

Thus, the present invention provides a pharmaceutical composition comprising the Ecklonia cava extract of the present invention, the fraction thereof, the fractional substance thereof, or the Ecklonia cava extract powder as an active ingredient for the treatment of at least one of diseases selected from the group consisting of such vascular diseases as vascular inflammatory diseases, vascular restenosis, vascular stenosis, atherosclerosis, myocardial infarction, angina pectoris, hypertension, hypertensive heart disease and peripheral vascular stenosis.

### Example 3: Isolation of PPB from Ecklonia cava extract

The following experiment was performed in order to separate and identify the compound represented by formula 1, pyrogallol-phloroglucinol-6,6-bieckol (PPB) from the Ecklonia cava extract.

### <3-1> Preparation of Ecklonia cava extract

Ecklonia cava powder provided from Aqua Green Technology (Jeju, Korea) was used for the preparation of the extract. 100 g of the Ecklonia cava powder was loaded in 4L of 50% ethanol, followed by extraction at room temperature (20□) for 24 hours. Centrifugation was performed and the supernatant was obtained. The supernatant was filtered with Watman filter paper (Whatman No. 4). The filtered extract was concentrated under reduced pressure to eliminate ethanol, followed by freeze-drying and pulverizing. As a result, the Ecklonia cava extract powder was prepared and stored at 4 .

### <3-2> Preparation of organic solvent fractions of Ecklonia cava extract

The Ecklonia cava prepared and freeze-dried in Example <3-1> was dissolved in distilled water, and solvent fractionation was performed by adding the organic solvents such as n-hexane, chloroform and ethyl acetate sequentially from nonpolar to polar organic solvent. The resulting product was concentrated under reduced pressure to obtain fractions, which was stored at 4 until it would be used.

### <3-3> HPLC of Ecklonia cava derived fractions

The Ecklonia cava ethyl acetate fraction (ECE) was analyzed by HPLC to identify the components of PPB.

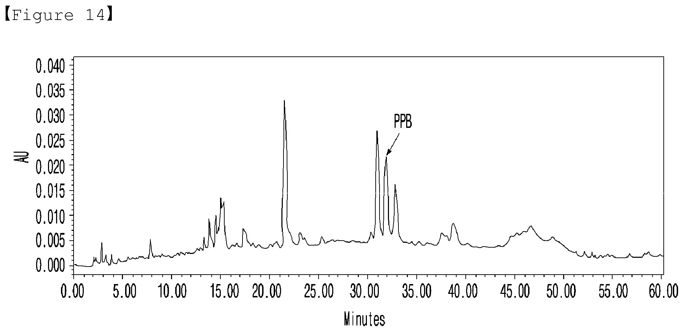

HPLC was performed by using gradient acetonitrile-water solvent system, for which 5 mg/mL of ECE sample was loaded on sunfire C18 column (3.5 µm 4.6 X 250). The moving phase was acetonitrile-water whose ratio was changed as follows. Particularly, acetonitrile and water were running as follows: 0 → 10 min (10:90 → 25:75 v/v), 10 → 40 min (25:75 → 35:65 v/v), 40 → 50 min (35:65 → 100:0 v/v) and 50 → 60 min (100:0 → 100:0 v/v). The flow rate was 1 mL/min. UV absorbance was measured at 254 nm, and the results are shown in Figure 14.

### <3-4> Isolation of PPB using centrifugal partition chromatography (CPC)

PPB was isolated from the Ecklonia cava extract ethyl acetate fraction (ECE) by CPC (centrifugal partition chromatography) as a single compound with the purity of 80-90%. For the CPC isolation, a two-phase solvent system that was not intermixed was used. The solvents used herein were n-hexane:ethyl acetate:methanol:water, which were mixed at the ratio of 2:8:3:7 in a separating funnel (4 L). After the mixture was stabilized in the separating funnel, the isolation was performed. The solvent in the upper layer was used as a stationary phase and the solvent in the lower layer was used as a moving phase.

For the isolation, a CPC column was filled with the stationary phase solvent. The moving phase solvent was flowed down to the column at the flow rate of 2 mL/min in a descending mode during the CPC rotor was rotating at 1,000 rpm until the moving phase solvent was fluidally stabilized (back pressure: 3.3 MPa) through the column.

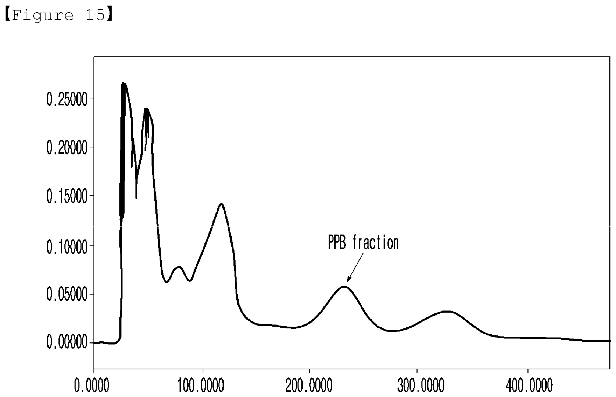

The ECE sample (500mg) was dissolved in a mixed solvent comprising the moving phase solvent and the stationary solvent at the ratio of 1:1, 3 mL each, which was injected through the injection valve. The fraction obtained from the CPC column was analyzed by UV at 290 nm. The fraction was collected in a 8 mL glass test tube by using a 6 mL fraction collector. The results are shown in Figure 15.

### <3-5> Analysis of CPC fraction by HPLC

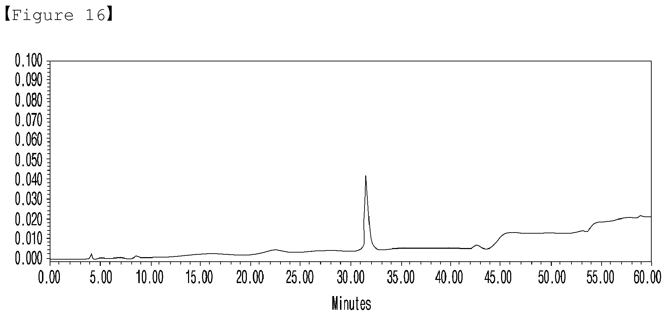

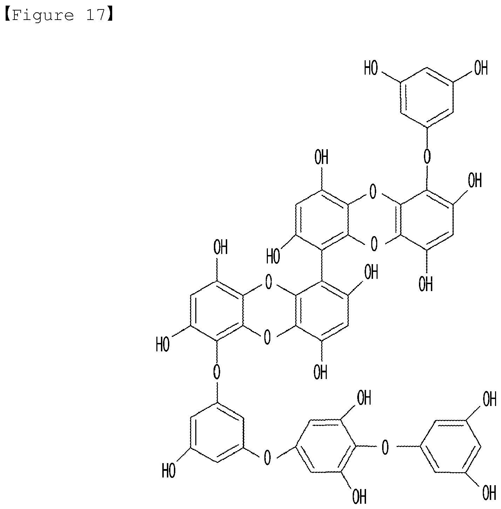

The fraction obtained by CPC in Example <3-4> proceeded to HPLC by the same manner as described in Example <3-3>. As a result, PPB with 90% purity was obtained. The results are shown in Figure 16 and the chemical structure of the isolated PPB is shown in Figure 17.

### Example 4: Evaluation of anti-obesity effect of PPB

The following experiment was performed to evaluate the anti-obesity effect of PPB isolated and identified in this invention.

### <4-1> Construction and treatment of obesity-induced animal model of ob/ob mouse

6-week-old male ob/ob mice were selected. Weight gain was induced by a general diet for 6 weeks. Then, the effect of the PPB prepared in Example 1 was investigated.

Particularly, the mice were adapted to the animal room environment for 1 week. Then, the mice were divided into two groups, which were fed with a general diet for 42 days (6 weeks). These two groups were the control (saline group) that was fed with a general diet and orally administered with saline and the experimental group (PPB group) that was fed with a general diet and orally administered with PPB at the dose of 2.5 mg/kg.

### <4-2> Measurement of fat cell size changes in obesity-induced animal model of ob/ob mouse

The adipose tissues were obtained from the mice of the control group and the PPB-treated experimental group prepared in Example <4-1>, and the changes of fat cell size were observed.

Particularly, the adipose tissues obtained from the mice of the control and experimental groups were made into a paraffin block, which was sectioned in the thickness of 7 µm, followed by hematoxylin- eosin staining (H&E staining). The stained tissues were photographed with Axio Imager Z1 upright microscopy system and the cell size was measured by using ImageJ 1.50i software.

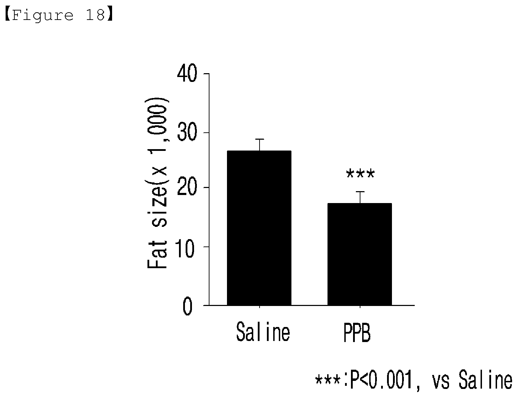

The changes in fat cell size induced by PPB in the obesity-induced animal model of ob/ob mouse are presented in Figure 18.

As shown in Figure 18, compared with the control group (saline group) in which the fat cell size was increased, the fat cell size was generally decreased in the experimental group (PPB group). The graph in the right indicates the numeric value of the fat cell size. As shown in this graph, the fat cell size was significantly decreased (p<0.005).

### <4-3> Measurement of fat accumulation in liver tissue in obesity-induced animal model of ob/ob mouse

The liver tissues were obtained from the mice of the control group and the experimental group prepared in Example <4-1>. The obtained liver tissues were made into a frozen block, which was sectioned in the thickness of 10 µm. The tissue sections were stained with 0.375% (w/v) Oil-red-O powder. The stained tissues were photographed with Axio Imager Z1 upright microscopy system and the fat accumulation area was measured by using ImageJ 1.50i software.

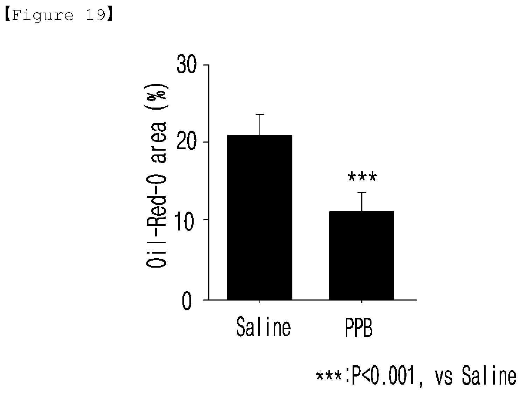

The changes of fat accumulation induced by PPB in the liver tissue of the obesity-induced animal model of ob/ob mouse are presented in Figure 19.

As shown in Figure 19, it was confirmed that the fat droplets turned red by Oil-red-O were reduced in the experimental group (PPB group), compared with the control group (saline group). The graph in the right indicates the fat accumulation as an area. As shown in this graph, the fat accumulation area was significantly decreased (p<0.005).

Therefore, the PPB compound was confirmed to have an anti-obesity effect. By such an effect, the PPB compound can be effectively used as an active ingredient of a pharmaceutical composition or a health functional food composition for the prevention or treatment of metabolic disease.

### Example 5: Evaluation of protective effect of PPB on vascular endothelial cells

The following experiment was performed in order to evaluate the protective effect of the PPB compound isolated and identified in this invention.

### <5-1> Mouse-derived vascular endothelial cell culture

Mouse-derived vascular endothelial cells (SVEC4-10) in DMEM (Dulbecco's Modified Eagle Medium) supplemented with 10% fetal bovine serum and 1% antibiotics (penicillin/streptomycin) were cultured in a 37 , 5% CO₂ incubator. To obtain sufficient cells, the cells were cultured in 100 Ø plastic dishes. When the cells were cultured until the bottom of the dish was covered 80% by the cells, the adhered cells were detached using trypsin/EDTA solution and then sub-cultured.

### <5-2> Construction and treatment of vascular endothelial cell death model

The mouse-derived vascular endothelial cells (SVEC4-10) obtained in Example <5-1> were treated with 0.25 mM palmitic acid, leading to the construction of a vascular endothelial cell death model (*in vitro*).

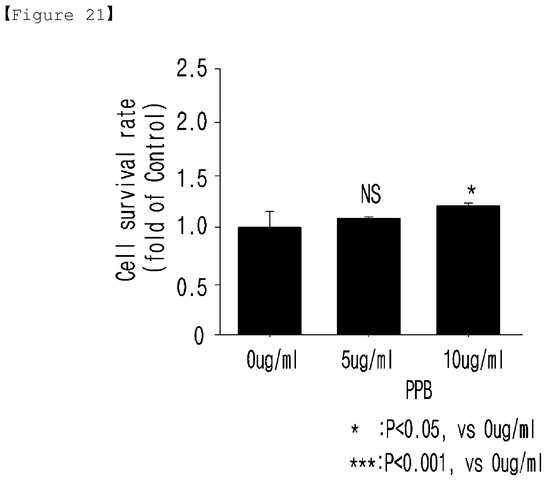

The cells were treated with PPB at different concentrations for 24 hours. 24 hours later, MTT assay was performed to confirm the number of survived cells. From the result, the effect of PPB on the survival of vascular endothelial cells in the presence of palmitic acid was analyzed. The results are shown in Figure 21.

### <5-3> Protective effect of PPB on cell death in vascular endothelial cell death model

As shown in Figure 21, when the control group non-treated with PPB and the experimental group treated with PPB at the dose of 5 ug/ml were compared, the number of survived cells was not much different between the two groups. However, the survival rate of vascular endothelial cells was significantly increased in the group treated with PPB at the dose of 10 ug/ml.

Therefore, it was confirmed that the compound represented by formula 1 had a protective effect on vascular endothelial cells. Thus, this compound can be used as an active ingredient of a pharmaceutical composition for the prevention or treatment or a health functional food composition for the prevention or improvement of vascular disease or thrombotic disease.

### Example 6: Evaluation of improvement effect of PPB on blood circulation

The following experiment was performed in order to evaluate the improvement effect of the PPB compound isolated and identified in this invention.

### <6-1> Measurement of plasminogen activator inhibitor type-1 (PAI-1) level in obesity-induced animal model of ob/ob mouse

Plasminogen activator inhibitor type-1 (PAI-1) is an important inhibitor of tissue plasminogen activator (tPA), the core serine protease responsible for plasminogenesis, and urokinase-type plasminogen activator (uPA).

PAI-1 regulates fibrinolysis by inhibiting plasminogen activation in the vascular region. Fibrinolysis is a strictly organized process for degrading the fibrin formed by activation of the coagulation step.

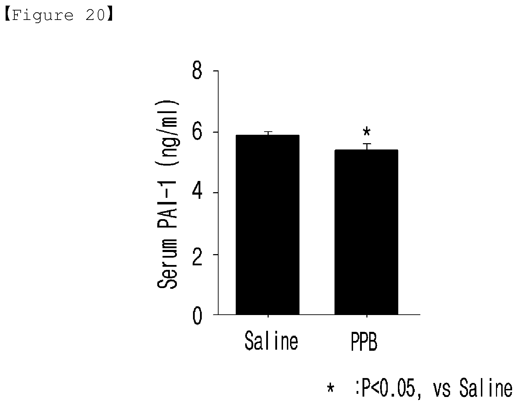

In order to measure PAI-1, the biomarker for improving blood circulation, blood was collected from the mouse, and the plasma PAI-1 level was measured by using an ELISA kit. The results are shown in Figure 20.

As shown in Figure 20, the blood PAl-1 level in the obesity-induced animal model of ob/ob mouse was compared between the control group treated with saline and the experimental group treated with PPB. As a result, the blood PAl-1 level was reduced in the experimental group, compared with the control group.

Therefore, it was confirmed that the generation of PAl-1 was inhibited by the injection of PPB.

As shown in the Experimental Examples above and Figures attached in this invention, PPB, the compound represented by formula 1 of the present invention, was confirmed to have the activities of reducing the levels of blood fibrinogen and plasminogen activator inhibitor type-1, increasing the expression of prostaglandin (PGI2), reducing the expressions of intracellular adhesion proteins (E-selectin, ICAM-1, VCAM-1, and vWF) and reducing the expression of endothelin-1 (ET-1).

Therefore, it was confirmed that this compound was efficient in suppressing thrombogenesis, preserving and improving blood vessel elasticity, suppressing abnormal proliferation of vascular visceral muscles, inhibiting endothelial cell damage, suppressing vascular outer wall fibrosis and preventing blood pressure increase.

Those skilled in the art will appreciate that the conceptions and specific embodiments disclosed in the foregoing description may be readily utilized as a basis for modifying or designing other embodiments for carrying out the same purposes of the present invention.

## Claims

1. A pharmaceutical composition comprising an Ecklonia cava extract as an active ingredient for use in the treatment of a vascular disease, wherein the Ecklonia cava extract is prepared by extraction using 45% ∼ 50% ethanol, and
the vascular disease is selected from the group consisting of vascular inflammatory diseases, vascular restenosis, vascular stenosis, arteriosclerosis, atherosclerosis, myocardial infarction, angina pectoris, hypertension, hypertensive heart disease and peripheral vascular stenosis.

2. The pharmaceutical composition for use in the treatment of a vascular disease according to claim 1, wherein the Ecklonia cava extract is prepared by extracting a whole Ecklonia cava plant or at least a part of the plant with 45% ∼ 50% ethanol.

3. The pharmaceutical composition for use in the treatment of a vascular disease according to any preceding claim, wherein the Ecklonia cava extract can prevent or treat a vascular disease by one or more activities selected from the group consisting of suppressing the migration or proliferation of vascular smooth muscle cells, increasing the expression of prostaglandin (PGI2), reducing the expressions of intracellular adhesion proteins (E-selectin, ICAM-1, VCAM-1, and vWF) and reducing the expression of endothelin-1 (ET-1).

4. The pharmaceutical composition for use in the treatment of a vascular disease according to any preceding claim, wherein the Ecklonia cava extract contains the compound represented by formula 1 below, the stereoisomer thereof or the pharmaceutically acceptable salt thereof as an active ingredient:

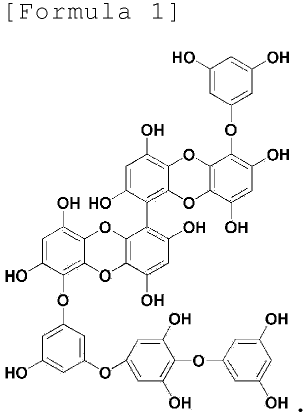

5. An Ecklonia cava extract for use in the treatment of a vascular disease, wherein the Ecklonia cava extract is prepared by extraction using 45% ∼ 50% ethanol, and
wherein the vascular disease is selected from the group consisting of vascular inflammatory diseases, vascular restenosis, vascular stenosis, arteriosclerosis, atherosclerosis, myocardial infarction, angina pectoris, hypertension, hypertensive heart disease and peripheral vascular stenosis.

6. An Ecklonia cava extract for use according to claim 5, wherein the Ecklonia cava extract is prepared by extracting the whole Ecklonia cava plant or at least a part of the plant with 45% ∼ 50% ethanol.

7. An Ecklonia cava extract for use according to any one of claims 5 to 6, wherein the Ecklonia cava extract can prevent or treat vascular disease by one or more activities selected from the group consisting of suppressing the migration or proliferation of vascular smooth muscle cells, increasing the expression of prostaglandin (PGI2), reducing the expressions of intracellular adhesion proteins (E-selectin, ICAM-1, VCAM-1, and vWF) and reducing the expression of endothelin-1 (ET-1).

8. An Ecklonia cava extract for use according to any one of claims 5 to 7, wherein the Ecklonia cava extract contains the compound represented by formula 1 below, the stereoisomer thereof or the pharmaceutically acceptable salt thereof as an active ingredient:

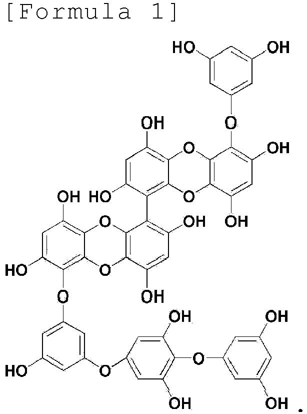

## Patentansprüche

1. Eine pharmazeutische Zusammensetzung, die einen Ecklonia-cava-Extrakt als einen Wirkstoff umfasst, zur Verwendung bei der Behandlung einer Gefäßerkrankung, wobei der Ecklonia-cava-Extrakt durch Extraktion unter Verwendung von 45% ∼ 50% Ethanol hergestellt wird, und
die Gefäßerkrankung ausgewählt ist aus der Gruppe bestehend aus vaskulären Entzündungserkrankungen, vaskulärer Restenose, vaskulärer Stenose, Arteriosklerose, Atherosklerose, Myokardinfarkt, Angina pectoris, Hypertonie, hypertensiver Herzkrankheit und peripherer vaskulärer Stenose.

2. Die pharmazeutische Zusammensetzung zur Verwendung bei der Behandlung einer Gefäßerkrankung gemäß Anspruch 1, wobei der Ecklonia-cava-Extrakt durch Extraktion einer ganzen Ecklonia-cava-Pflanze oder wenigstens eines Teils der Pflanze mit 45% ∼ 50% Ethanol hergestellt wird.

3. Die pharmazeutische Zusammensetzung zur Verwendung bei der Behandlung einer Gefäßerkrankung gemäß einem vorhergehenden Anspruch, wobei der Ecklonia-cava-Extrakt eine Gefäßerkrankung durch eine oder mehrere Aktivitäten, ausgewählt aus der Gruppe bestehend aus Unterdrücken der Migration oder Proliferation von vaskulären glatten Muskelzellen, Steigern der Expression von Prostaglandin (PGI2), Verringern der Expressionen von intrazellulären Adhäsionsproteinen (E-Selectin, ICAM-1, VCAM-1 und vWF) und Verringern der Expression von Endothelin-1 (ET-1), verhindern oder behandeln kann.

4. Die pharmazeutische Zusammensetzung zur Verwendung bei der Behandlung einer Gefäßerkrankung gemäß einem vorhergehenden Anspruch, wobei der Ecklonia-cava-Extrakt die durch die nachstehende Formel 1 dargestellte Verbindung, das Stereoisomer davon oder das pharmazeutisch annehmbare Salz davon als einen Wirkstoff enthält:

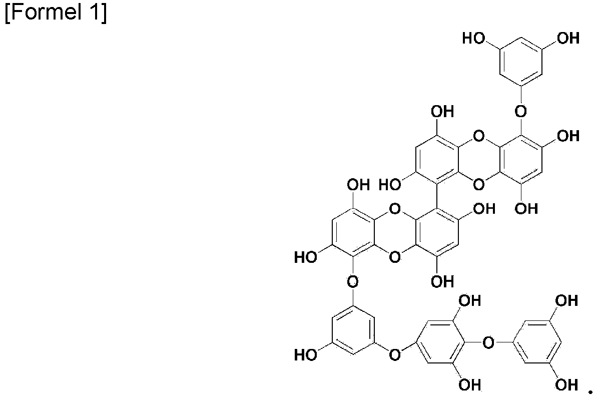

5. Ein Ecklonia-cava-Extrakt zur Verwendung bei der Behandlung einer Gefäßerkrankung, wobei der Ecklonia-cava-Extrakt durch Extraktion unter Verwendung von 45% - 50% Ethanol hergestellt wird, und
wobei die Gefäßerkrankung ausgewählt ist aus der Gruppe bestehend aus vaskulären Entzündungserkrankungen, vaskulärer Restenose, vaskulärer Stenose, Arteriosklerose, Atherosklerose, Myokardinfarkt, Angina pectoris, Hypertonie, hypertensiver Herzkrankheit und peripherer vaskulärer Stenose.

6. Ein Ecklonia-cava-Extrakt zur Verwendung gemäß Anspruch 5, wobei der Ecklonia-cava-Extrakt durch Extraktion der ganzen Ecklonia-cava-Pflanze oder wenigstens eines Teils der Pflanze mit 45% ∼ 50% Ethanol hergestellt wird.

7. Ein Ecklonia-cava-Extrakt zur Verwendung gemäß einem der Ansprüche 5 bis 6, wobei der Ecklonia-cava-Extrakt eine Gefäßerkrankung durch eine oder mehrere Aktivitäten, ausgewählt aus der Gruppe bestehend aus Unterdrücken der Migration oder Proliferation von vaskulären glatten Muskelzellen, Steigern der Expression von Prostaglandin (PGI2), Verringern der Expressionen von intrazellulären Adhäsionsproteinen (E-Selectin, ICAM-1, VCAM-1 und vWF) und Verringern der Expression von Endothelin-1 (ET-1), verhindern oder behandeln kann.

8. Ein Ecklonia-cava-Extrakt zur Verwendung gemäß einem der Ansprüche 5 bis 7, wobei der Ecklonia-cava-Extrakt die durch die nachstehende Formel 1 dargestellte Verbindung, das Stereoisomer davon oder das pharmazeutisch annehmbare Salz davon als einen Wirkstoff enthält:

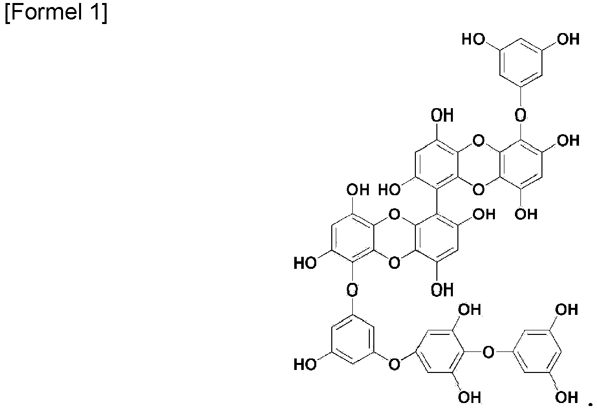

## Revendications

1. Composition pharmaceutique comprenant un extrait d'Ecklonia cava en tant que principe actif pour une utilisation dans le traitement d'une maladie vasculaire, dans laquelle l'extrait d'Ecklonia cava est préparé par extraction utilisant 45 à 50 % d'éthanol, et
la maladie vasculaire est choisie dans le groupe constitué par les maladies inflammatoires vasculaires, une resténose vasculaire, une sténose vasculaire, une artériosclérose, une athérosclérose, un infarctus du myocarde, une angine de poitrine, une hypertension, une maladie cardiaque hypertensive et une sténose vasculaire périphérique.

2. Composition pharmaceutique pour une utilisation dans le traitement d'une maladie vasculaire selon la revendication 1, dans laquelle l'extrait d'Ecklonia cava est préparé par extraction d'un plant d'Ecklonia cava entier ou d'au moins une partie de la plante avec 45 à 50 % d'éthanol.

3. Composition pharmaceutique pour une utilisation dans le traitement d'une maladie vasculaire selon l'une quelconque des revendications précédentes, dans laquelle l'extrait d'Ecklonia cava peut prévenir ou traiter une maladie vasculaire par une ou plusieurs activités choisies dans le groupe constitué par une suppression de la migration ou de la prolifération de cellules musculaires lisses vasculaires, une augmentation de l'expression de prostaglandine (PGI2), une réduction de l'expression de protéines d'adhésion intracellulaire (E-sélectine, ICAM-1, VCAM-1 et vWF), et une réduction de l'expression d'endothéline-1 (ET-1).

4. Composition pharmaceutique pour une utilisation dans le traitement d'une maladie vasculaire selon l'une quelconque des revendications précédentes, dans laquelle l'extrait d'Ecklonia cava contient, en tant que principe actif, le composé représenté par la formule 1 ci-dessous, son stéréoisomère ou son sel pharmaceutiquement acceptable :

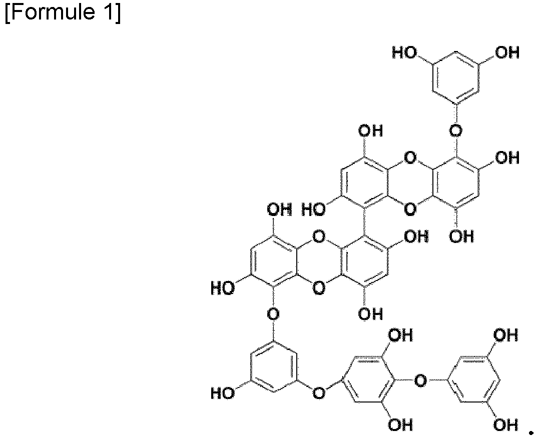

5. Extrait d'Ecklonia cava pour une utilisation dans le traitement d'une maladie vasculaire, lequel extrait d'Ecklonia cava est préparé par extraction utilisant 45 à 50 % d'éthanol, et
dans lequel la maladie vasculaire est choisie dans le groupe constitué par les maladies inflammatoires vasculaires, une resténose vasculaire, une sténose vasculaire, une artériosclérose, une athérosclérose, un infarctus du myocarde, une angine de poitrine, une hypertension, une maladie cardiaque hypertensive et une sténose vasculaire périphérique.

6. Extrait d'Ecklonia cava pour une utilisation selon la revendication 5, lequel extrait d'Ecklonia cava est préparé par extraction d'un plant d'Ecklonia cava entier ou d'au moins une partie de la plante avec 45 à 50 % d'éthanol.

7. Extrait d'Ecklonia cava pour une utilisation selon l'une quelconque des revendications 5 et 6, lequel extrait d'Ecklonia cava peut prévenir ou traiter une maladie vasculaire par une ou plusieurs activités choisies dans le groupe constitué par une suppression de la migration ou de la prolifération de cellules musculaires lisses vasculaires, une augmentation de l'expression de prostaglandine (PGI2), une réduction de l'expression de protéines d'adhésion intracellulaire (E-sélectine, ICAM-1, VCAM-1 et vWF), et une réduction de l'expression d'endothéline-1 (ET-1).

8. Extrait d'Ecklonia cava pour une utilisation selon l'une quelconque des revendications 5 à 7, lequel extrait d'Ecklonia cava contient, en tant que principe actif, le composé représenté par la formule 1 ci-dessous, son stéréoisomère ou son sel pharmaceutiquement acceptable :
